# EUROPEAN PATENT SPECIFICATION

(11) **EP 1 345 951 B1**
(45) Date of publication and mention of the grant of the patent: **18.08.2004**
(21) Application number: 01999570.3
(22) Date of filing: 29.11.2001
(51) Int. Cl.: C07F 19/00, A61K 31/295, A61P 35/04, C07F 17/02

(54) **A SUBSTANCE OR COMPOSITION FOR THE TREATMENT OF CANCER**
EINE SUBSTANZ ODER EINE ZUSAMMENSETZUNG ZUR KREBSBEHANDLUNG
SUBSTANCE OU COMPOSITION DE TRAITEMENT ANTICANCEREUX

(30) Priority: 04.12.2000 ZA 200007167
(43) Date of publication of application: 24.09.2003
(73) Proprietor: University of Pretoria, Pretoria 0002, Gauteng Province (ZA); University of the Free State, 9300 Bloemfontein (ZA)
(72) Inventor: SWARTS, Johannes Christiaan, Dan Pienaar, 9300 Bloemfontein (ZA); MEDLEN, Constance Elizabeth, Rieetondale, 0084 Pretoria (ZA)
(74) Representative: Hübner, Gerd, Dipl.-Phys.
(86) International application number: PCT/IB2001/002258
(87) International publication number: WO 2002/046203

(56) References cited:
- WOISETSCHLAGER, OLIVER E. ET AL: "Hydrocarbon-bridged metal complexes. Part 49. Coordination chemistry of bis(ferrocenyl)-substituted 1,3-diketonates with ruthenium, rhodium, iridium, and palladium" Z. ANORG. ALLG. CHEM. , vol. 626, no. 3, 2000, pages 766-774, XP008001637
- CULLEN, WILLIAM R. ET AL: "Rhodium(I) complexes of.beta.-diketonates and related ligands as hydrosilylation catalysts" J. ORGANOMET. CHEM. (1989), 370(1-3), 141-54 , vol. 370, no. 1-3, 1989, pages 141-154, XP000033877
- PATENT ABSTRACTS OF JAPAN vol. 1997, no. 07, 31 July 1997 (1997-07-31) & JP 09 067386 A (KAO CORP), 11 March 1997 (1997-03-11)
- HAUSER ET AL.: "Benzoylations of both methylketone groups of bisacetylferrocene with methyl benzoate and alkali amides to form the bis-beta-diketone. Certain derivatives." J. ORG. CHEM., vol. 23, 1958, pages 1142-1146, XP002197289
- HAUSER ET AL.: "Acylations of bisacetylferrocenes with esters by potassium amide to form bis-beta-diketones. Consideration of mechanism." J. ORG. CHEM, vol. 26, 1961, pages 1030-1034, XP002197290
- DU PLESSIS, W. C. ET AL: "Cyclic voltammetry of ferrocene-containing.beta.-diketones as a tool to obtain group electronegativities. The structure of 3-ferrocenoyl-1,1,1-trifluoro-2-hydroxypro p-2-ene" CANADIAN JOURNAL OF CHEMISTRY, vol. 77, no. 3, 1999, pages 378-386, XP001073559
- PLESSIS ET AL.: "beta-diketonates containing a ferrocenyl group: synthesis, structural aspects, pKa values, group electronegativities and complexation with Rhodium" J. CHEM. SOC., DALTON TRANS., 1998, pages 2507-2514, XP002197291 cited in the application
- DATABASE CA [Online] CHEMICAL ABSTRACTS SERVICE, COLUMBUS, OHIO, US; YANG, XUEJIN ET AL: "Analysis of organometallic compounds. XII. Reversed-phase high-performance liquid chromatography of sulfonyl-, carbonyl-, and pyrazolyl-substituted ferrocene derivatives" retrieved from STN Database accession no. 109:66135 XP002197292 & SEPU (1988), 6(1), 54-6 ,
- BISCHOFF, H. ET AL: "Efficacy of.beta.-diketonato complexes of titanium, zirconium, and hafnium against chemically induced autochthonous colonic tumors in rats" J. CANCER RES. CLIN. ONCOL., vol. 113, no. 5, 1987, pages 446-450, XP008001636

## Description

THIS INVENTION relates to the treatment of cancer.

In particular, the invention relates to a substance or composition for use in the treatment of cancer, to the use of a substance or composition in the preparation of a medicament for the treatment of cancer, to a method of treating cancer, to a substance or composition for use in a method of sensitising cells to radiation, to the use of a substance or composition in the preparation of a medicament for use in sensitising cells to radiation, to a method of sensitising cells to radiation and to a metallocenyl β diketone.

Woisetschlager *et al* (*Z Anorg Allg Chem, Vol 626, No. 3, 2000, 776 - 774)* discloses the reaction of the enolates of diferrocenoylmethane and of spacer bridged bis-,tris- and tetrakis(ferrocenoyl)-1,3-diketones with chlorobridged compounds to produce a series of mono-,bis,tris-and tetrakis(chelate) complexes. Cullen and Wickenheiser *(J Organomet Chem, 1989, 370 (1 - 3), 141 - 154)* describes the use of rhodium complexes as catalysts for the hydrosilylation of PhMeCO with Ph₂SiH₂. *Patent Abstracts of Japan, Vol 1997, No. 7, 31 July 1997 (Kao Corp)* describes a method of producing chiral 1,3-diketone compounds with a high optical purity which can provide complexes with various metals useful as asymmetric synthetic reaction catalysts by reacting acetyl ferrocenes with trifluoroethoxycarbonylferrocenes. The chiral 1,3-diketone compounds are substituted diferrocenoylmethanes. Hauser and Cain *(J Org Chem, Vol 23, 1958, 1142 - 1146*) describe the benzoylation of both methyl ketone groups of bisacetylferrocene with excess methyl benzoate and alkali amide to form the bis-β-diketone. The bis-β-diketone was converted to the bispyrazole and to a copper chelate. Cain, Mashburn and Hauser *(J Org Chem Vol 26, 1961, 1030* - *1034*) describe the acylation at both methyl groups of bisacetylferrocene with esters by potassium amide in liquid ammonia to form bis-β-diketones. The bis-β-diketones were reacted with an excess of hydrazine to form bis-pyrazoles. Du Plessis *et al (Canadian Journal of Chemistry, Vol 77, No. 3, 1999, 378* - *386*) describe the cyclic voltammetry of ferrocene-containing β-diketones as a tool to obtain group electronegativities. The compounds investigated included ferrocenoyltrifluoroacetone (Hfctfa), ferrocenoyltrichloroacetone (Hfctca), ferrocenoylacetone (Hfca), benzoylferrocenoylmethane (Hbfcm) and diferrocenoylmethane (Hdfcm). Yang *et al (Chemical Abstracts Service, 109: 66135)* describes the analysis of a number of sulphonyl-,carbonyl- and pyrazolyl-substituted ferrocene derivatives by reverse-phase HPLC using a C₁₈-bonded stationary phase. Bishoff *et al (J Cancer Res Clin Oncol*, *Vol 113, No. 5, 1987, 446 - 450)* disclose the testing of bis-β-diketenato complexes of titanium, zirconium and hafnium against colorectal tumours in rats. Of the compounds tested, budotitane was the most effective in terms of decreasing tumour weight and number and increasing the lifespan of the treated animals. The therapeutic efficiency was superior to that of 5-fluorouracil.

According to a first aspect of the invention there is provided a substance or composition for use in the treatment of cancer, the substance or composition including
(a) at least one compound which is selected from metallocenyl β-diketones of the general formula Mc-CO-CZ₁Z₂-CO-R in which Mc is selected from Fc (ferrocenyl), Rc (ruthenocenyl) and Oc (osmocenyl), R is H, alkyl or aryl, haloalkyl, Mc and Z₁ and Z₂ are independently H, alkyl aryl or substituted alkyl or ferrocenyl, or
(b) at least one compound which is selected from the enol forms of the β-diketones and metal complexes of the β-diketones of the general formula M(β-diketonato)A¹, M(β-diketonato)A¹A², M(β-diketonato)A¹B¹B², M(β-diketonato)B¹B² and M(β-diketonato)B¹B²B³B⁴ in which
β-diketonato is Mc-CO-CZ₁Z₂-CO-R as described above,
M is selected from Rh and Ir,
A¹ and A² are the same or different and are selected from cyclic dienes having 6 - 8 carbons, or linear alkenes having 2-7 carbons
B¹, B², B³ and B⁴ are the same or different and are selected from CO, P(R¹R² R³), P(OR¹)(OR²)(OR³), R⁴ and X in which R¹,R², R³ and R⁴ are the same or different and are independently selected from alkyl, phenyl and ferrocenyl, and X is a halide or a pseudohalide, and the method including the step of administering to a person or animal in need of treatment a therapeutically effective dose of the substance or composition.

Z₁ and Z₂ may be selected from haloalkyl and benzyl. R may be selected from CF₃, CCl₃, CH₃, H, Ph (phenyl) and Mc. The halide may be F, Cl, Br or I. The pseudohatide may be N₃, NCO or SCN.

The compound may be selected from the group consisting of [M(β-diketonato)(cod)], [M(β-diketonato)(CO)₂], [M(β-diketonato)(CO)(PR²₃)], [M(β-diketonato){P-(OR²)₃}₂] [M(β-diketonato)(CO)₂(R³)(X)] or its acyl isomer [M(β-diketonato)(CO)(COR³)(X)], [M(β-diketonato)(CO)(PR²₃)(R³)(X)] or its acyl isomer [M(β-diketonato)(PR²₃)(COR³)(X)], [M(β-diketonato){P-(OR²)₃}₂(R³)(X)] and [M(β-diketonato)(cod)(R³)(X)], in which M is Rh or Ir, cod is 1,5-cyclooctadiene, (β-diketonato) is (McCOCHCOR) in which R is selected from CF₃, CCl₃, CH₃, H, phenyl and Mc, R² is alkyl, phenyl, ferrocenyl and combinations thereof, R³ is alkyl, phenyl or ferrocenyl , and X is a halide or pseudohalide.

Preferred compounds in accordance with the invention are set out in the following compound list.

### COMPOUND LIST

(1) ferrocenoylacetaldehyde, (Hfch),
(2) ferrocenoyltrichloroacetone, (Hfctca),
(3) (η⁴-1,5-cyclooctadiene)(1-ferroceno-1,3-propanedionato-(4) κ²O,O')rhodium(1), [Rh(FcH)(cod)],
(4) (η⁴-1,5-cyclooctadiene)(1,3-diferrocenyl-1,3-propanedionato-κ²O,O')rhodium(1) [Rh(dfcm)(cod)],
(5) (η⁴-1,5-cyclooctadiene)(1-ferrocenyl-3-phenyl-1,3-propanedionato-κ²O,O')rhodium(1) [Rh(bfcm)(cod)],
(6) (η⁴-1,5-cyclooctadiene)(1-ferrocenyl-1,3-butanedionato-κ²O,O')rhodium(1), [Rh(fca)(cod)],
(7) (η⁴-1,5-cyclooctadiene)(1-ferrocenyl-4,4,4-trichloro-1,3-butanedionato-κ²O,O')rhodium(1), [Rh(fctca)(cod)],
(8) (η⁴-1,5-cyclooctadiene)(1-ferrocenyl-4,4,4-trifluooro-1,3-butanedionato-κ²O,O')rhodium(1), [Rh(fctfa)(cod)],
(9) diferrocenoylmethane, (Hdfcm),
(10) ferrocenoyltrifluoroacetone, (Hfctfa),
(11) (η⁴-1,5-cyclooctadiene)(1-ferrocenyl-4,4,4-trifluoro-1,3-butanedionato-κ²O,O')iridium(1), [Ir(fctfa)(cod)],
(12) (η⁴-1,5-cyclooctadiene)(1-ferrocenyl-1,3-butanedionato-κ²O,O')iridium(1), [Ir(fca)(cod)],
(13) (η⁴-1,5-cyclooctadiene)(1-ferrocenyl-3-phenyl-1,3-propanedionato-κ²O,O')iridium(1), [Ir(bfcm)(cod)],
(14) benzoylferrocenoylmethane, (Hbfcm),
(15) ferrocenoylacetone, (Hfca),
(16) (η⁴-1,5-cyclooctadiene)(1-ferrocenyl-1,3-propanedionato-κ²O,O')iridium(1), [Ir(fch)(cod)].
(17) (η⁴-1,5-cyclooctadiene)(1-ruthenocenyl-1,3-propanedionato-κ²O,O')rhodium(1), [Rh(RcH)(cod)],
(18) (η⁴-1,5-cyclooctadiene)(1,3-diruthenocenyl-1,3-propanedionato-κ²O,O')rhodium(1) [Rh(drcm)(cod)],
(19) (η⁴-1,5-cyclooctadiene)(1-ruthenocenyl-3-phenyl-1,3-propanedionato-κ²O,O')rhodium(1) [Rh(brcm)(cod)],
(20) (η⁴-1,5-cyclooctadiene)(1-ruthenocenyl-1,3-butanedionato-κ²O,O')rhodium(1), [Rh(rca)(cod)],
(21)(η⁴-1,5-cyclooctadiene)(1-ruthenocenyl-4,4,4-trichloro-1,3-butanedionato-κ²O,O')rhodium(1), [Rh(rctca)(cod)],
(22) (η⁴-1,5-cyclooctadiene)(1-ruthenocenyl-4,4,4-trifluooro-1,3-butanedionato-κ²O,O')rhodium(1), [Rh(rctfa)(cod)],
(23) (η⁴-1,5-cyclooctadiene)(1-osmocenyl-1,3-propanedionato-κ²O,O')rhodium(1), [Rh(OcH)(cod)],
(24) (η⁴-1,5-cyclooctadiene)(1,3-diosmocenyl-1,3-propanedionato-κ²O,O')rhodium(1) [Rh(docm)(cod)],
(25) (η⁴-1,5-cyclooctadiene)(1-osmocenyl-3-phenyl-1,3-propanedionato-κ²O,O')rhodium(1) [Rh(bocm)(cod)],
(26) (η⁴-1,5-cyclooctadiene)(1-osmocenyl-1,3-butanedionato-κ²O,O')rhodium(1), [Rh(oca)(cod)],
(27) (η⁴-1,5-cyclooctadiene)(1-osmocenyl-4,4,4-trichloro-1,3-butanedionato-κ²O,O')rhodium(1), [Rh(octca)(cod)],
(28) (η⁴-1,5-cyclooctadiene)(1-osmocenyl-4,4,4-trifluooro-1,3-butanedionato-κ²O,O')rhodium(1), [Rh(octfa)(cod)],
(29) (η⁴-1,5-cyclooctadiene)(1-ferrocenyl-3-ruthenocenyl-1,3-propanedionato-κ²O,O')rhodium(1) [Rh(fcrcm)(cod)],
(30) (η⁴-1,5-cyclooctadiene)(1-ferrocenyl-3-osmocenyl-1,3-propanedionato-κ²O,O')rhodium(1) [Rh(fcocm)(cod)],
(31) (η⁴-1,5-cyclooctadiene)(1-osmocenyl-3-ruthenocenyl-1,3-propanedionato-κ²O,O')rhodium(1) [Rh(ocrcm)(cod)],
(32) (η⁴-1,5-cyclooctadiene)(1,3-pentanedionato-κ²O,O')rhodium(1) [Rh(acac)(cod)],
(33) 1-ruthenocenyl-1,3-propanedione = HRcH,
(34) 1,3-diruthenocenyl-1,3-propanedione = Hdrcm,
(35) 1-ruthenocenyl-3-phenyl-1,3-propanedione = Hbrcm,
(36) ruthenocenoylacetone = 1-ruthenocenyl-1,3-butanedione = Hrca,
(37) ruthenocenoyltrichloroacetone = 1-ruthenocenyl-4,4,4-trichloro-1,3-butanedione = Hrctca,
(38) ruthenocenoyltrifluoroacetone = 1-ruthenocenyl-4,4,4-trifluoro-1,3-butanedione = Hrctfa,
(39) 1-osmocenyl-1,3-propanedione = HOcH
(40) 1,3-diosmocenyl -1,3-propanedione = Hdocm
(41) 1-osmocenyl-3-phenyl-1,3-propanedione = Hbocm,
(42) osmocenoylacetone = 1-osmocenyl-1,3-butanedione = Hoca,
(43) osmocenoyltrichloroacetone = 1-osmocenyl-4,4,4-trichloro-1,3-butanedione = Hoctca,
(44)osmocenoyltrifluoroacetone = 1-osmocenyl-4,4,4-trifluoro-1,3-butanedione = Hoctfa,
(45) ferrocenoylosmocenoylmethane = 1 ferrocenyl-3-osmocenyl-1,3-propanedione = Hfcocm,
(46) osmocenoylruthenocenoylmethane = 1-osmocenyl-3-ruthenocenyl-1,3-propanedione = Hrcocm,
(47) ferrocenoylruthenocenoylmethane = 1-ferrocenyl-3-ruthenocenyl-1,3-propanedione = Hfcrcm,
(48) (η⁴-1,5-cyclooctadiene)(1-ferrocenyl-3-ruthenocenyl-1,3-propanedionato-κ²O,O')rhodium(1) [Rh(fcrcm)(cod)],
(49) (η⁴-1,5-cyclooctadiene)(1-ferrocenyl-3-osmocenyl-1,3-propanedionato-κ²O,O')rhodium(1) [Rh(fcocm)(cod)]

### Examples of compounds in accordance with the invention are set out in the Appendix.

According to another aspect of the invention there is provided the use of a substance or composition in the preparation of a medicament for the treatment of cancer, the substance or composition including
(a) at least one compound which is selected from metallocenyl β-diketones of the general formula Mc-CO-CZ₁Z₂-CO-R in which Mc is selected from Fc (ferrocenyl), Rc (ruthenocenyl) and Oc (osmocenyl), R is H, alkyl, haloalkyl, Mc or aryl and Z₁ and Z₂ are independently H, alkyl, aryl or substituted alkyl or ferrocenyl, or
(b) at least one compound which is selected from the enol forms of the β-diketones and metal complexes of the β-diketones of the general formula M(β-diketonato)A¹, M(β-diketonato)A¹A², M(β-diketonato)A¹B¹B², M(β-diketonato)B¹B² and M(β-diketonato)B¹B²B³B⁴ in which
β-diketonato is Mc-CO-CZ₁Z₂-CO-R as described above
M is selected from Rh and Ir,
A¹ and A² are the same or different and are selected from cyclic dienes having 6 - 8 carbons or linear alkenes having 2 - 7 carbons
B¹, B², B³ and B⁴ are the same or different and are selected from CO, P(R¹R² R³), P(OR¹)(OR²)(OR³), R⁴ and X in which R¹,R², R³ and R⁴ are the same or different and are independently selected from alkyl, phenyl and ferrocenyl, and X is a halide or a pseudohalide.

Z₁ and Z₂ may be selected from haloalkyl and benzyl. R may be selected from CF₃, CCl₃, CH₃, H, Ph (phenyl) and Mc. The halide may be selected from F, Cl, Br and I. The pseudohalide may be selected from N₃, NCO and SCN.

The compound may be selected from the group consisting of [M(β-diketonato)(cod)], (M(β-diketonato)(CO)₂], [M(β-diketonato)(CO)(PR²₃)], [M(β-diketonato){P-(OR²)₃}₂] [M(β-diketonato)(CO)₂(R³)(X)] or its acyl isomer, [M(β-diketonato)(CO)(COR³)(X)], [M(β-diketonato)(CO)(PR²₃)(R³)(X)] or its acyl isomer, [M(β-diketonato)(PR²₃)(COR³)(X)], [M(β-diketonato){P-(OR²)₃}₂(R³)(X)] and [M(β-diketonato)(cod)(R³)(X)], in which M is Rh or Ir, cod is 1,5-cyclooctadiene, (β-diketonato) is (McCOCHCOR) in which R is selected from CF₃, CCl₃, CH₃, H, phenyl and Mc, R² is alkyl, phenyl, ferrocenyl and combinations thereof, R³ is alkyl, phenyl or ferrocenyl, and X is a halide or pseudohalide.

Preferred compounds in accordance with the invention are set out in the Compound List.

According to another aspect of the invention there is provided a method of treating cancer, the method including the step of administering to a person or animal in need of treatment a therapeutically effective dose of a substance or composition which includes
(a) at least one compound which is selected from metallocenyl β-diketones of the general formula Mc-CO-CZ₁Z₂-CO-R in which Mc is selected from Mc (ferrocenyl), Rc (ruthenocenyl) and Oc (osmocenyl), R is H, alkyl, haloalkyl, Mc or aryl and Z₁ and Z₂ are independently H, alkyl, aryl or substituted alkyl or ferrocenyl, or
(b) at least one compound which is selected from the enol forms of the β-diketones and metal complexes of the β-diketones of the general formula M(β-diketonato)A¹, M(β-diketonato)A¹A², M(β-diketonato)A¹B¹B², M(β-diketonato)B¹B² and M(β-diketonato)B¹B²B³B⁴ in which
β-diketonato is Mc-CO-CZ₁Z₂-CO-R as described above
M is selected from Rh and Ir,
A¹ and A² are the same or different and are selected from cyclic dienes having 6 - 8 carbons or linear alkenes having 2 - 7 carbons
B¹, B², B³ and B⁴ are the same or different and are selected from CO, P(R¹R² R³), P(OR¹)(OR²)(OR³), R⁴ and X in which R¹,R², R³ and R⁴ are the same or different and are independently selected from alkyl, phenyl and ferrocenyl, and X is a halide or a pseudohalide.

Z₁ and Z₂ may be selected from haloalkyl and benzyl. R may be selected from CF₃, CCl₃, CH₃, H, Ph (phenyl) and Mc. The halide may be F, Cl, Br or I. The pseudohalide may be N₃, NCO or SCN.

The compound may be selected from the group consisting of [M(β-diketonato)(cod)], [M(β-diketonato)(CO)₂], [M(β-diketonato)(CO)(PR²₃)], [M(β-diketonato){P-(OR²)₃}₂] (M(β-diketonato)(CO)₂(R³)(X)] or its acyl isomer [M(β-diketonato)(CO)(COR³)(X)], [M(β-diketonato)(CO)(PR²₃)(R³)(X)] or its acyl isomer [M(β-diketonato)(PR²₃)(COR³)(X)], [M(β-diketonato){P-(OR²)₃}₂(R³)(X)] and [M(β-diketonato)(cod)(R³)(X)], in which M is Rh or Ir, cod is 1,5-cyclooctadiene, (β-diketonato) is (McCOCHCOR) in which R is selected from CF₃, CCl₃, CH₃, H, phenyl and Mc, R² is alkyl, phenyl, ferrocenyl and combinations thereof, R³ is alkyl, phenyl or ferrocenyl , and X is a halide or pseudohalide.

Preferred compounds in accordance with the invention are set out in the Compound List.

The compounds of the invention are of particular use in the treatment of a patient with cancer which has built up, or could build up, resistance to other therapeutically active substances. The compounds of the invention are also of particular use in the treatment of a patient with cancer which has built up, or could build up, resistance to radiotherapy, and can be administered before, together with or after radiotherapy.

According to another aspect of the invention there is provided a substance or composition for use in a method of sensitising cells to radiation, the substance of composition including
(a) at least one compound which is selected from metallocenyl β-diketones of the general formula Mc-CO-CZ₁Z₂-CO-R in which Mc is selected from Fc (ferrocenyl), Rc (ruthenocenyl) and Oc (osmocenyl), R is H, alkyl, haloalkyl, Mc or aryl and Z₁ and Z₂ are independently H, alkyl, aryl or substituted alkyl or ferrocenyl, or
(b) at least one compound which is selected from the enol forms of the β-diketones and metal complexes of the β-diketones of the general formula M(β-diketonato)A¹, M(β-diketonato)A¹A², M(β-diketonato)A¹B¹B², M(β-diketonato)B¹B² and M(β-diketonato)B¹B²B³B⁴ in which
β-diketonato is Mc-CO-CZ₁Z₂-CO-R as described above
M is selected from Rh and Ir,
A¹ and A² are the same or different and are selected from cyclic dienes having 6 - 8 carbons or linear alkenes having 2 - 7 carbons
B¹, B², B³ and B⁴ are the same or different and are selected from CO, P(R¹R² R³), P(OR¹)(OR²)(OR³), R⁴ and X in which R¹,R², R³ and R⁴ are the same or different and are independently selected from alkyl, phenyl and ferrocenyl, and X is a halide or a pseudohalide.

Z₁ and Z₂ may be selected from haloalkyl and benzyl. R may be selected from CF₃, CCl₃, CH₃, H, Ph (phenyl) and Mc. The halide may be F, Cl, Br or I. The pseudohalide may be N₃, NCO or SCN.

The compound may be selected from the group consisting of [M(β-diketonato)(cod)], [M(β-diketonato)(CO)₂], [M(β-diketonato)(CO)(PR²₃)], [M(β-diketonato){P-(OR²)₃}₂] [M(β-diketonato)(CO)₂(R³)(X)] or its acyl isomer [M(β-diketonato)(CO)(COR³)(X)], [M(β-diketonato)(CO)(PR²₃)(R³)(X)] or its acyl isomer [M(β-diketonato)(PR²₃)(COR³)(X)], [M(β-diketonato){P-(OR²)₃}₂(R³)(X)] and [M(β-diketonato)(cod)(R³)(X)], in which M is Rh or Ir, cod is 1,5-cyclooctadiene, (β-diketonato) is (McCOCHCOR) in which R is selected from CF₃, CCl₃, CH₃, H, phenyl and Mc, R² is alkyl, phenyl, ferrocenyl and combinations thereof, R³ is alkyl, phenyl or ferrocenyl, and X is a halide or pseudohalide.

Preferred compounds in accordance with the invention are set out in the Compound List.

According to another aspect of the invention there is provided the use of a substance or composition in the preparation of a medicament for use in sensitising cells to radiation, the substance or composition including
(a) at least one compound which is selected from metallocenyl β-diketones of the general formula Mc-CO-CZ₁Z₂-CO-R in which Mc is selected from Fc (ferrocenyl), Rc (ruthenocenyl) and Oc (osmocenyl), R is H, alkyl, haloalkyl, Mc or aryl and Z₁ and Z₂ are independently H, alkyl, aryl or substituted alkyl or ferrocenyl, or
(b) at least one compound which is selected from enol forms of the β-diketones and metal complexes of the β-diketones of the general formula M(β-diketonato)A¹, M(β-diketonato)A¹A², M(β-diketonato)A¹B¹B², M(β-diketonato)B¹B² and M(β-diketonato)B¹B²B³B⁴ in which
β-diketonato is Mc-CO-CZ₁Z₂-CO-R as described above
M is selected from Rh and Ir,
A¹ and A² are the same or different and are selected from cyclic dienes having 6 - 8 carbons or linear alkenes having 2 - 7 carbons
B¹, B², B³ and B⁴ are the same or different and are selected from CO, P(R¹R² R³), P(OR¹(OR²)(OR³), R⁴ and X in which R¹,R², R³ and R⁴ are the same or different and are independently selected from alkyl, phenyl and ferrocenyl, and X is a halide or a pseudohalide.

Z₁ and Z₂ may be selected from haloalkyl and benzyl. R may be selected from CF₃, CCl₃, CH₃, H, Ph (phenyl) and Mc. The halide may be F, Cl, Br or I. The pseudohalide may be N₃, NCO or SCN.

The compound may be selected from the group consisting of [M(β-diketonato)(cod)], [M(β-diketonato)(CO)₂], [M(β-diketonato)(CO)(PR²₃)], [M(β-diketonato){P-(OR²)₃}₂] [M(β-diketonato)(CO)₂(R³)(X)] or its acyl isomer [M(β-diketonato)(CO)(COR³)(X)], [M(β-diketonato)(CO)(PR²₃)(R³)(X)] or its acyl isomer [M(β-diketonato)(PR²₃)(COR³)(X)], [M(β-diketonato){P-(OR²)₃}₂(R³)(X)] and [M(β-diketonato)(cod)(R³)(X)], in which M is Rh or Ir, cod is 1,5-cyclooctadiene, (β-diketonato) is (McCOCHCOR) in which R is selected from CF₃, CCl₃, CH₃, H, phenyl and Mc, R² is alkyl, phenyl, ferrocenyl and combinations thereof, R³ is alkyl, phenyl or ferrocenyl, and X is a halide or pseudohalide.

Preferred compounds in accordance with the invention include (η⁴-1,5-cyclooctadiene)(1,3-pentanedionato-κ²O,O')rhodium(1) [Rh(acac)(cod)], and the compounds in the Compound List.

According to another aspect of the invention there is provided a method of sensitising cells to radiation, the method including the step of exposing the cells before, during or after irradiation, to
(a) at least one compound which is selected from metallocenyl β-diketones of the general formula Mc-CO-CZ₁Z₂-CO-R in which Mc is selected from Fc (ferrocenyl), Rc (ruthenocenyl) and Oc (osmocenyl), R is H, alkyl, haloalkyl, Mc or aryl and Z₁ and Z₂ are independently H, alkyl, aryl or substituted alkyl or ferrocenyl, or
(b) at least one compound which is selected from the enol forms of the β-diketones and metal complexes of the β-diketones of the general formula M(β-diketonato)A¹, M(β-diketonato)A¹A², M(β-diketonato)A¹B¹B², M(β-diketonato)B¹B² and M(β-diketonato)B¹B²B³B⁴ in which
β-diketonato is Mc-CO-CZ₁Z₂-CO-R as described above
M is selected from Rh and Ir,
A¹and A² are the same or different and are selected from cyclic dienes having 6 - 8 carbons or linear alkenes having 2 - 7 carbons
B¹, B², B³ and B⁴ are the same or different and are selected from CO, P(R¹R² R³), P(OR¹)(OR²)(OR³), R⁴ and X in which R¹,R², R³ and R⁴ are the same or different and are independently selected from alkyl, phenyl and ferrocenyl, and X is a halide or a pseudohalide, and the method including the step of administering to a person or animal in need of treatment a therapeutically effective dose of the substance or composition.

Z₁ and Z₂ may be selected from haloalkyl and benzyl. R may be selected from CF₃, CCl₃, CH₃, H, Ph (phenyl) and Mc. The halide may be F, Cl, Br or I. The pseudohalide may be N₃, NCO or SCN.

The compound may be selected from the group consisting of [M(β-diketonato)(cod)], [M(β-diketonato)(CO)₂], [M(β-diketonato)(CO)(PR²₃)], [M(β-diketonato){P-(OR²)₃}₂] [M(β-diketonato)(CO)₂(R³)(X)] or its acyl isomer [M(β-diketonato)(CO)(COR³)(X)], [M(β-diketonato)(CO)(PR²₃)(R³)(X)] or its acyl isomer [M(β-diketonato)(PR²₃)(COR³)(X)], [M(β-diketonato){P-(OR²)₃}₂(R³)(X)] and [M(β-diketonato)(cod)(R³)(X)], in which M is Rh or Ir, cod is 1,5-cyclooctadiene, (β-diketonato) is (McCOCHCOR) in which R is selected from CF₃, CCl₃, CH₃, H, phenyl and Mc, R² is alkyl, phenyl, ferrocenyl and combinations thereof, R³ is alkyl, phenyl or ferrocenyl, and X is a halide or pseudohalide.

Preferred compounds in accordance with the invention include (η⁴-1,5-cyclooctadiene)(1,3-pentanedionato-κ²O,O')rhodium(1) [Rh(acac)(cod)], and the compounds in the Compound List.

The substance or composition will, in particular, be used for sensitizing cells to radiation under hypoxic conditions.

According to another aspect of the invention there is provided a compound which is
(a) selected from metallocenyl β-diketones of the general formula Mc-CO-CZ₁Z₂-CO-R in which Mc is selected from Fc (ferrocenyl), Rc (ruthenocenyl) and Oc (osmocenyl), R is H, alkyl, haloalkyl, Mc or aryl and Z₁ and Z₂ are independently H, alkyl, aryl or substituted alkyl or ferrocenyl, or
(b) selected from the enol forms of the β-diketones and metal complexes of the β-diketones of the general formula M(β-diketonato)A¹, M(β-diketonato)A¹A², M(β-diketonato)A¹B¹B², M(β-diketonato)B¹B² and M(β-diketonato)B¹B²B³B⁴ in which
β-diketonato is Mc-CO-CZ₁Z₂-CO-R as described above,
M is selected from Rh and Ir,
A¹ and A² are the same or different and are selected from cyclic dienes having 6 - 8 carbons, or linear alkenes having 2-7 carbons
B¹, B², B³ and B⁴ are the same or different and are selected from CO, P(R¹R² R³), P(OR¹)(OR²)(OR³), R⁴ and X in which R¹,R², R³ and R⁴ are the same or different and are independently selected from alkyl, phenyl and ferrocenyl, and X is a halide or a pseudohalide,
with the proviso that the compound may not be
(1) ferrocenoyltrichloroacetone, (Hfctca),
(2) (η⁴-1,5-cyclooctadiene)(1,3-diferrocenyl-1,3-propanedionato-κ²O,O')rhodium(1) [Rh(dfcm)(cod)],
(3) (η⁴-1,5-cyclooctadiene)(1-ferrocenyl-3-phenyl-1,3-propanedionato-κ²O,O')rhodium(1) [Rh(bfcm)(cod)],
(4) (η⁴-1,5-cyclooctadiene)(1-ferrocenyl-1,3-butanedionato-κ²O,O')rhodium(1), [Rh(fca)(cod)],
(5) (η⁴-1,5-cyclooctadiene)(1-ferrocenyl-4,4,4-trichloro-1,3-butanedionato-κ²O,O')rhodium(1), [Rh(fctca)(cod)],
(6) (η⁴-1,5-cyclooctadiene)(1-ferrocenyl-4,4,4-trifluooro-1,3-butanedionato-κ²O,O')rhodium(1), [Rh(fctfa)(cod)],
(7) diferrocenoylmethane, (Hdfcm),
(8) ferrocenoyltrifluoroacetone, (Hfctfa),
(9) benzoylferrocenoylmethane, (Hbfcm),
(10) ferrocenoylacetone, (Hfca),
(11) (η⁴-1,5-cyclooctadiene)(1,3-pentanedionato-κ²O,O')rhodium(1) [Rh(acac)(cod)],
(12) (η²-1,2-ethene)₂(1-ferrocenyl-1,3-butanedionato-k²O,O')rhodium(1),[Rh(fca)(CH₂CH₂)₂]

Z₁ and Z₂ may be selected from haloalkyl and benzyl. R may be selected from CF₃, CCl₃, CH₃, H, Ph (phenyl) and Mc. The halide may be F, Cl, Br or I. The pseudohalide may be N₃, NCO or SCN.

The compound may be selected from the group consisting of [M(β-diketonato)(cod)], [M(β-diketonato)(CO)₂], [M(β-diketonato)(CO)(PR²₃)], [M(β-diketonato){P-(OR²)₃}₂] [M(β-diketonato)(CO)₂(R³)(X)] or its acyl isomer [M(β-diketonato)(CO)(COR³)(X)], [M(β-diketonato)(CO)(PR²₃)(R³)(X)] or its acyl isomer [M(β-diketonato)(PR²₃)(COR³)(X)], [M(β-diketonato){P-(OR²)₃}₂(R³)(X)] and [M(β-diketonato)(cod)(R³)(X)], in which M is Rh or Ir, cod is 1,5-cyclooctadiene, (β-diketonato) is (McCOCHCOR) in which R is selected from CF₃, CCl₃, CH₃, H, phenyl and Mc, R² is alkyl, phenyl, ferrocenyl and combinations thereof, R³ is alkyl, phenyl or ferrocenyl, and X is a halide or pseudohalide.

Preferred compounds are set out in the Compound List.

The invention is now described, by way of example, with reference to the accompanying Examples and the Figures in which
Figure 1 is a graph showing the growth of various cancer cell lines and PHA-stimulated human lymphocytes as a function of Hfctfa concentration;
Figure 2 is a graph of the growth of various cancer cell lines and PHA-stimulated human lymphocites as a function of [Rh(fctfa)(cod)] concentration;
Figure 3 is a graph of the growth of various cancer cell lines and PHA-stimulated human lymphocites as a function of the concentration of [Rh(fctca)(cod)] concentration;
Figure 4 is a graph of survival fraction as a function of radiation dosage;
Figure 5 is a graph of the survival fraction of CHO cells following irradiation under hypoxic conditions in the presence of µM [Rh(fctca)(cod)] as a function of radiation dosage; and

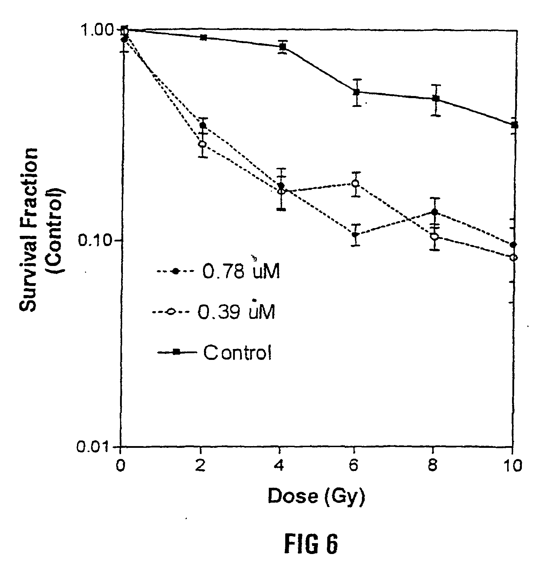

Figure 6 is a graph of the survival fraction of CHO cells following irradiation under hypoxic conditions in the presence of µM [Rh(fctfa)(cod)] as a function of radiation dosage; and

The examples describe *in vitro* studies of metallocene-containing β-diketones of the type Mc-CO-CH₂CZ₁Z₂-CO-R in which Mc is as hereinbefore described R is CF₃ (Hfctfa)], CCl₃ (Hfctca), CH₃ (Hfca), H (Hfch), Ph (Hbfcm) and Fc (Hdfcm) and their rhodium complexes Rh(β-diketonato)(cod),

### EXAMPLE 1

### Effects of ferrocene-containing β-diketones and their rhodium complexes on the growth of cancer cell lines

In these experiments cancer cell lines were cultured in standard tissue culture medium supplemented with 10% fetal calf serum (FCS) at 37°C in an atmosphere of 5% CO₂ in 96 well round bottom microtitre plates. The cultures were treated with either ferrocene-containing β-diketones or their rhodium complexes at varying concentrations for 72 to 96h, and the extent of cell growth was assayed by MTT [3-(4,5-dimethylthiazol-2-yl)-2,5-diphenyl tetrazolium bromide] reactivity, which detects only viable cells.

The sensitivities of the two cancer cell lines HeLa, which is a sensitive human cervix epitheloid carcinoma cell line, and CoLo 320DM, which is an intrinsically multidrug resistant human colon adeno-carcinoma cell line, to 13 of the complexes of the invention was tested.

Eight of the more active complexes were also tested on the two cell lines COR L23, which is a sensitive human lung large cell carcinoma cell line, and COR L23/CPR, which is a variant of COR L23 and which is resistant to malphalan and other platinum compounds.

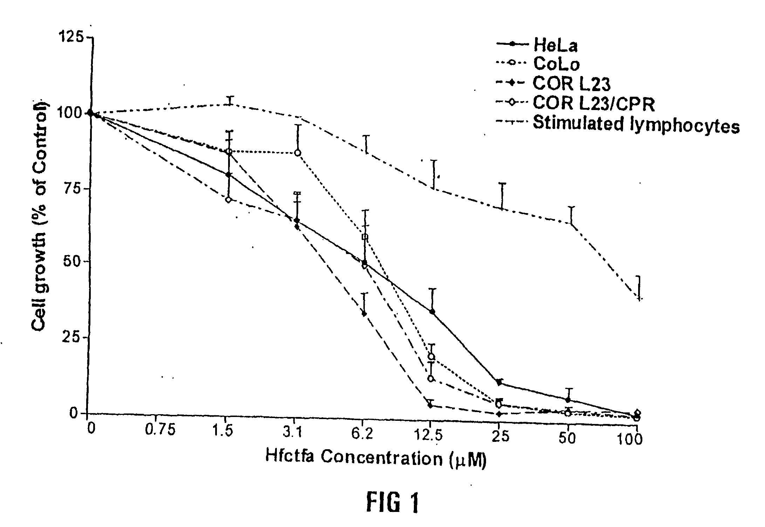

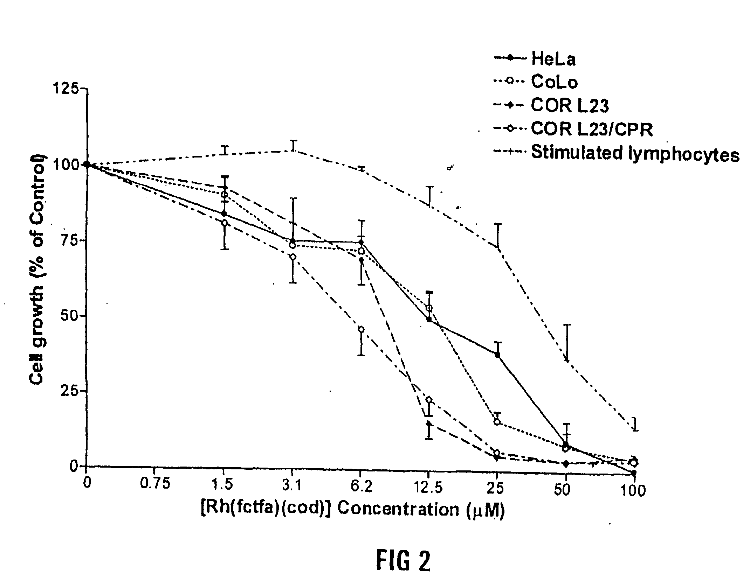

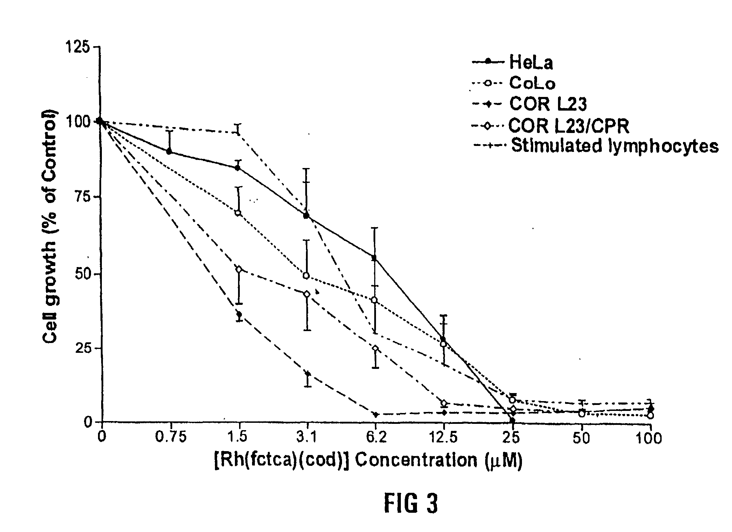

All the ferrocene-containing β-diketones, as well as most of their rhodium-cod complexes inhibited the growth of all of the cancer cell lines tested, including those which are drug resistant, at concentrations <100µM (Table 1). The most active compound amongst the ferrocene complexes was Hfctfa (Table 1, Figure 1) whereas the two most active rhodium-ferrocene complexes were [Rh(fctfa)(cod)] and [Rh(fctca)(cod)] (Table 1, Figures 2 and 3)

**TABLE 1**

| **Chemosensitivity of cancer cell lines and PHA-stimulated human lymphocytes to ferrocene, rhodium-ferrocene complexes and rhodium ruthenocene complexes** **IC 50 (µM)*of the following cell lines for complexes** | | | | | |
|---|---|---|---|---|---|
| **Complexes** | **HeLa** | **CoLo 320DM** | **COR L23** | **COR L23/CPR** | **PHA- stimulated lymphocytes** |
| **Ferrocene:** | | | | | |
| Hfch | 73.4 | 80.8 | Nd** | Nd | Nd |
| Hfctca | 37.7 | 28.4 | 12.5 | 20.1 | 67.5 |
| Hdfcm | 54.4 | 64.3 | 75.4 | 74.4 | >100 |
| Hfctfa | 6.8 | 7.3 | 4.5 | 6.3 | 83.1 |
| Hbfcm | 54.2 | 85.1 | 66.8 | 80.4 | >100 |
| Hfca | 66.6 | 57.1 | Nd | Nd | Nd |

| **Rhodium-Ferrocene:** | | | | | |
|---|---|---|---|---|---|
| Rh(acac)(cod) | 97.3 | >100 | Nd | Nd | Nd |
| Rh(FcH)(cod) | 62.4 | 35.4 | Nd | Nd | Nd |
| Rh(dfcm)(cod) | 18.4 | 70.8 | 19.9 | 30.8 | 42.2 |
| Rh(bfcm)(cod) | 28.3 | 87.1 | 22.5 | 22.0 | 97.2 |
| Rh(fca)(cod) | 64.4 | 56.6 | Nd | Nd | Nd |
| Rh(fctca)(cod) | 7.9 | 3.0 | 1.3 | 2.0 | 4.7 |
| Rh(fctfa)(cod) | 12.5 | 13.4 | 8.5 | 5.8 | 41.6 |
| [Rh(RcH)(cod)] | 52.007 | 68.803 | 33.973 | 39.615 | |
| [Rh(drcm)(cod)] | NT | NT | NT | NT | |
| [Rh(brcm)(cod)] | 16.218 | 20.435 | 15.783 | 14.099 | |
| [Rh(rca)(cod)] | 4.863 | 3 | 0.18 | 2.74 | |
| [Rh(rctca)(cod)] | NT | NT | NT | NT | |
| [Rh(rctfa)(cod)] | 54.742 | 67.438 | 49.207 | 47.993 | |
| [Rh(fcrcm)(cod)] | 17.125 | 29.207 | 10.101 | 20.108 | |

| | | | | | |
|---|---|---|---|---|---|
| * Data from 3-4 experiments are expressed as the mean drug concentration (µM)-causing 50% cell killing. | | | | | |
| ** not done | | | | | |

### EXAMPLE 2

### Effects of ferrocene-containing β-diketones and their rhodium complexes on the proliferation of human lymphocytes

In these experiments suspensions of purified human mononuclear leukocytes were cultured in RPMI medium supplemented with 10% fetal calf serum (FCS) at 37°C in an atmosphere of 5% CO₂ in 96 well round bottom microtitre plates. To some of the wells a mitogen (phytohaemagglutinin, PHA) was added at a concentration of 2.5µg/ml. The cultures were treated with either ferrocene-containing β-diketones or their rhodium complexes at varying concentrations for 72h, and the extent of cell growth was assayed by MTT reactivity.

The ferrocene complexes which were tested inhibited 50% of the growth of PHA-stimulated lymphocyte cultures only at concentrations of 67.5µM and higher (Table 1). The lymphocyte cultures were, however, more sensitive to the rhodium-ferrocene complexes tested with [Rh(bfcm)(cod)] the least toxic to stimulated lymphocytes (Table 1).

The ideal anti-tumor agent should be an agent with high activity against cancer cells, including drug resistant cells, and low activity against stimulated normal human lymphocytes. Hfctfa possesses the highest tumor specificity and is eight times less active against normal human lymphocytes than tumor cells. This compound would therefore be a possible candidate for the treatment of various cancers, including multidrug resistant cancers. Another possible candidate, [Rh(fctfa)(cod)], is 4-5 times more active against tumor cells than against stimulated human lymphocytes.

### EXAMPLE 3

### Effects of rhodium-ferrocene complexes on the survival of Chinese hamster ovary (CHO) cells after irradiation under aerobic and hypoxic conditions.

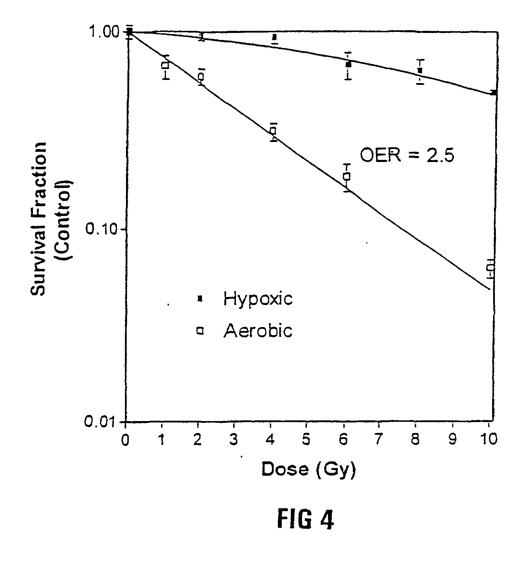

CHO cells were treated in glass test tubes with non-toxic concentrations (0.39µM and 0.78µM) of the two rhodium-ferrocene complexes [Rh(fctca)(cod)] and [Rh(fctfa)(cod)] and irradiated with an 8 MV photon beam in a modular incubator chamber with a 2cm tissue equivalent wax buildup (8MV dₘₐₓ) under aerobic and hypoxic conditions. An oxygen enhancement ratio of 2.5 (Figure 4) was obtained indicating that the method used to establish a hypoxic cellular environment was highly effective.

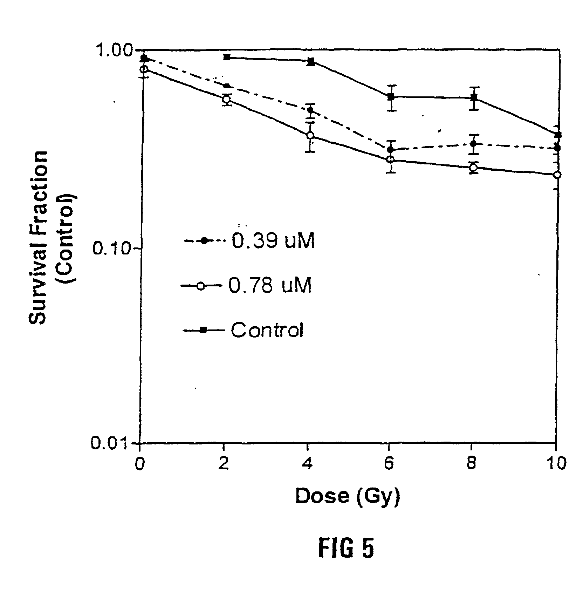

Neither of the two rhodium complexes tested increased the sensitivity of CHO cells to irradiation in an aerobic environment (results not shown). However, both complexes increased the sensitivity of CHO cells under hypoxic conditions (Figures 4 and 5). A dose-modifying factor of 2.9 was obtained under hypoxic conditions for 0.78µM [Rh(fctfa)(cod)] (Table 2) and 1.9 for 0.39µM [Rh(fctca)(cod)] (Table 3). The dose-modifying factors were calculated as the ratio of mean inactivation doses [calculated from the respective inactivation parameters obtained from the linear quadratic fit of the cell growth fraction (S)]. Published dose modifying factors (DMFs) measured for cisplatin range from 1.2-2 *(ChibberR, Stratford IJ, O'Neill P, Sheldon PW, Ahmed I, Lee B. 1985. The interaction between radiation and complexes of cis-Pt (II) and Rh (II): Studied at the molecular level.* ***Int J Radiat Biol 48****:513-524; Van De Vaart, PJM, Klaren, HM, Hofland I, Begg AC. 1997. Oral platinum* analogue *JM216,* a *radiosensitizer in oxic murine cells.* ***Int J Radiat Biol 72****(6):675-683)*.

These results demonstrate that the two rhodium-ferrocene complexes, [Rh(fctfa)(cod)] and [Rh(fctca)(cod)] compare favorably with cisplatin as inducers of hypoxia selective radiosensitization in rapidly proliferating CHO cells at submicromolar concentrations, suggesting that these complexes may be clinically useful in combination with radiation in the treatment of cancers. Initial tests with Hfctfa and Hfctca were also promising.

**TABLE 2**

| | | |
|---|---|---|
| **Mean inactivation doses calculated from the response of Chinese hamster ovary cells following treatment with radiation and/or different concentrations of [Rh(fctfa)(cod)] under hypoxic conditions. Dose modifying factors are stated as the ratio of mean inactivation doses.** | | |

| **Treatment** | **Mean Inactivation Dose (Gy)** | **Dose Modifying Factor (DMF)** |
|---|---|---|
| Radiation (Hypoxia) | 6.44 | |
| Rh(fctfa)(cod) 0.39µM | 2.41 | 2.6 |
| Rh(fctfa)(cod) 0.78µM | 2.19 | 2.9 |

**TABLE 3**

| | | |
|---|---|---|
| **Mean inactivation doses calculated from the response of Chinese hamster ovary cells following treatment with radiation and/or different concentrations of [Rh(fctca)(cod)] under hypoxic conditions. Dose modifying factors are stated as the ratio of mean inactivation doses.** | | |

| **Treatment** | **Mean Inactivation Dose (Gy)** | **Dose Modifying Factor (DMF)** |
|---|---|---|
| Radiation | 7.16 | |
| (Hypoxia) Rh(fctca)(cod) | 3.73 | 1.9 |
| 0.39µM Rh(fctca)(cod) | 4.46 | 1.6 |
| 0.78µM | | |

### EXAMPLE 4

### SYNTHESIS OF THE METALLOCENE COMPOUNDS

The synthesis of the new ferrocene-containing, ruthenocene-containing and osmocene-containing betadiketones can all be achieved by Claisen condensation of acetylferrocene, acetylruthenocene or acetylosmocene with the appropriate ester under the influence of lithium diisopropylamide. A selected example of each of these classes of compounds is set out below. Co-ordination with rhodium is also demonstrated

### Betadiketone synthesis

### The ferrocene series: The synthesis of 1-ferrocenyl-1,3-propanedione, HFcH

To an ice-cool solution of acetylferrocene (2.28 g, 10 mmol) in dry, air free THF (18 ml) was added under nitrogen 5.5 ml of a 2.0 mol dm⁻³ THF solution of lithium diisopropyl amide. Stirring continued for 20 minutes during which time a brick red precipitate formed. A solution of dry, cold methyl formiate (0.6 g, 10 mmol) in THF (1 ml) was then added and stirring continued overnight. After 16 hours, diethyl ether (15 ml) was added to the reaction mixture, the precipitate filtered, washed with ether and air dried. The precipitate was then suspended in 0.5 mol dm⁻³ HCl and extracted with ether, the ether extracts dried with MgSO₄, and the solvent removed to liberate HFcH in 35 yield. The crude product can be crystallised from hexane/ether (1:1).

### The ruthenocene series: The synthesis of 1-ruthenocenyl-1,3-butanedione, Hrca.

To an ice-cool solution of acetylruthenocene (0.6 g, 2.2 mmol) in dry, air free THF (4 ml) was added under nitrogen 1.1 ml of a 1.6 mol dm⁻³ THF solution of lithium diisopropyl amide. Stirring continued for 20 minutes during which time a cream precipitate formed. A solution of dry ethyl acetate (x ml, 2.2 mmol) in THF (1 ml) was then added and stirring continued overnight. After 16 hours, diethyl ether (15 ml) was added to the reaction mixture, the precipitate filtered, washed with ether and air dried. The precipitate was then suspended in 0.5 mol dm⁻³ HCl and extracted with ether, the ether extracts dried with MgSO₄, and the solvent removed to liberate Hrca in 30-40% yield. The crude product can be purified further by column chromatography on Kieselgel utilising ether/hexane (3:2) as eluent.

### The osmocene series: The synthesis 1-osmocenyl-4,4,4-trifluoro-1,3-butanedione, Hoctfa.

To an ice-cool solution of acetylosmocene (0.25 g, 0.69 mmol) in dry, air free THF (1 ml) was added under nitrogen 0.35 ml of a 2 mol dm⁻³ THF solution of lithium diisopropyl amide. Stirring continued for 20 minutes during which time a cream precipitate formed. A solution of dry ethyl trifluoroacetate (0.1 g, 0.69 mmol) in THF (0.6 ml) was then added and stirring continued overnight. After 16 hours, diethyl ether (10 ml) was added to the reaction mixture, the precipitate filtered, washed with ether and air dried. The precipitate was then suspended in 0.5 mol dm⁻³ HCl and extracted with ether, the ether extracts dried with MgSO₄, and the solvent removed to liberate Hoctfa in 30 % yield. The crude product can be crystallised from hexane/ether (1:1).

### The mixed metallocene series: The synthesis of 1-ferrocenyl-3-ruthenocenylpropanedianato, Hfcrcm.

To an ice-cool solution of acetylruthenocene (0.6 g, 2.2 mmol) in dry, air free THF (5 ml) was added under nitrogen 1.1 ml of a 2.0 mol dm⁻³ THF solution of lithium diisopropyl amide. Stirring continued for 20 minutes during which time a cream precipitate formed. A solution of dry methyl ferrocenoate (0.537 g, 2.2 mmol) in THF (1 ml) was then added and stirring continued overnight. After 16 hours, diethyl ether (15 ml) was added to the reaction mixture, the precipitate filtered, washed with ether and air dried. The precipitate was then suspended in 0.5 mol dm⁻³ HCl and extracted with ether, the ether extracts dried with MgSO₄, and the solvent removed to liberate Hfcrcm in 30-40% yield. The crude product can be purified further by column chromatography on Kieselgel utilising ether/hexane (3:2) as eluent.

### Rhodium co-ordination

### The synthesis of (η⁴-1,5-cyclooctadiene)(1-ferrocenyl-1,3-propanedionato κ²O,O')rhodium(I), [Rh(FcH)(cod)].

To a stirred solution of [Rh₂Cl₂(cod)₂] (0.5g, 1 mmol) in 6 ml DMF was added 1-ferrocenyl-1,3-propanedione (0.512 g, 2 mmol). After 5 minutes, the crude product was precipitated with an excess of water, filtered and dissolved in ether. The ether solution was washed with water, dried with magnesium sulphate. After solvent removal the residue was crystallised from cold ether/hexane mixtures starting with a ratio of 1:1. Pure crystals of [Rh(FcH)(cod)] were filtered in 65% yield from the mother liquor.

### The synthesis of (η⁴-1,5-cyclooctadiene)(1-ruthenocenyl-1,3-butanedionato-κ²O,O')rhodium(I), [Rh(rca)(cod)]

To a stirred solution of [Rh₂Cl₂(cod)₂] (0.5g, 1 mmol) in 6 ml DMF was added 1-ruthenocenyl-1,3-butanedione (0.631 g, 2 mmol). After 5 minutes, the crude product was precipitated with an excess of water, filtered and dissolved in ether. The ether solution was washed with water, dried with magnesium sulphate and the solvent removed. The crude product was then crystallised from cold ether/hexane mixtures starting with a ratio of 1:1. Pure crystals of [Rh(rca)(cod)] were filtered in 75% yield from the mother liquor.

### The synthesis of (η⁴-1,5-cyclooctadiene)(1-osmocenyl-4,4,4-trifluoro-1,3-propanedionato-κ²O,O'))rhodium(I), [Rh(octfa)(cod)]

To a stirred solution of [Rh₂Cl₂(cod)₂] (0.5g, 1 mmol) in 6 ml DMF was added 1-osmocenyl-4,4,4-trifluoro-1,3-butanedione (0.917 g, 2 mmol). After 5 minutes, the crude product was precipitated with an excess of water, filtered and dissolved in ether. The ether solution was washed with water, dried with magnesium sulphate. After solvent removal, the residue was crystallised from cold ether/hexane mixtures starting with a ratio of 1:1. Pure crystals of [Rh(octfa)(cod)] were filtered in 55% yield from the mother liquor.

### The synthesis of (η⁴-1,5-cyclooctadiene)(1-ferrocenyl-3-ruthenocenyl-1,3-propanedionato-κ²O,O')rhodium(I), [Rh(fcrcm)(cod)]

To a stirred solution of [Rh₂Cl₂(cod)₂] (0.5g, 1 mmol) in 6 ml DMF was added 1-ferrocenyl-1,3-propanedione (0.97 g, 2 mmol). After 5 minutes, the crude product was precipitated with an excess of water, filtered and dissolved in ether. The ether solution was washed with water, dried with magnesium sulphate and the crude product crystallised after solvent removal from cold ether/hexane mixtures starting with a ratio of 1:1. Pure crystals of [Rh(fcrcm)(cod)] were obtained in 65% yield from the mother liquor.

### DISCUSSION

Cisplatin is one of the most widely used drugs for the chemotherapy of cancer *(Muggia FM. 1991; Introduction: Cisplatin update.* ***Seminars in Oncology 18:****1-4)*. However, this compound has serious side effects including nausea, vomiting and nephrotoxicity *(Rosenberg B.1985;* ***Fundamental studies with cisplatin.*** *Cancer* ***55****:2303-2316)* all of which are dose limiting. The search for novel organometallic complexes exhibiting higher antineoplastic activity and decreased side-effects, has stimulated the interest of several investigators and many reports are available on the antineoplastic activity of transitional metal complexes. Among them some rhodium complexes appear to be promising antitumor agents *(Bear JL, Gray HB, Rainen L, Chang I-M, Howard R, Serio G, Kimball AP*. *1975. Interaction of Rhodium II carboxylates with molecules of biologic importance; Cancer Chemother Rep 59: 611-620; Fiamiani V, Ainis T, Cavallaro A, Piraino P. 1990; Antitumor effects of the new rhodium (II) complex: Rh*_{*2*}*(Form)*_{*2*}*(O*_{*2*}*CCF*_{*3*}*)* _{*2*}*(H*_{*2*}*O)* _{*2*} *(Form=N,N'-di-p-tolylformamidinate;* ***J Chemother 2:****319-326*. *Sartori R, Rencoret G, Rencoret G, Mora A, Perez C, Pastene R, Sariego R, Moya SA. 1997; The novel use of Rh (I) complexes with naphthyridine ligands and poly (oxyethylene) as antitumorals*. ***Anti-Cancer Drugs 6:****87-92)*. Some of these rhodium complexes, despite the heavy-metal character of rhodium, seem to show no nephrotoxicity *(Kopf-Maier P. 1994. Complexes of metals other than platinum as antitumoral agents*. ***Eur J Clin Pharmacol 47:*** *1-16; Craciunescu DG, Scaria V, Furlani A, Papaionnou A, Iglesias EP, Alonso MP. 1991. Pharmacological and toxicological studies on new Rh (I) organometalic complexes.* ***In Vivo 5:*** *329-332J.*

The presence of hypoxic cells, resistant to radiotherapy as a consequence of the rapid metabolism of oxygen in tumor tissue, is a limiting factor in the successful treatment of tumors by radiation. Sensitization of radioresistant tumors can be achieved by the use of chemical radiosensitizers in combination with conventional radiotherapy.

The Applicant has found that metallocene-containing β-diketones of the type Mc-CO-CH₂-CO-R in which Mc is selected from Fc (ferrocenyl), Rc (ruthenocenyl) and Oc (osmocenyl), and R is CF₃, CCl₃, CH₃, H, Ph (phenyl) or Fc, the enol forms of the β-diketones and rhodium and iridium complexes of the β-diketones previously untested in biological systems inhibit the growth of various cancer cells, including platinum resistant strains and multidrug resistant cancer cells. Furthermore, some of these complexes have also been found to sensitize cells to radiation under hypoxic conditions.

The synthesis of Fc-CO-CH₂-CO-R, in which R is CF₃, CCl₃, CH₃, Ph and Fc, as well as Rh(β-diketonato)(cod) has been described *(Du Plessis WC, Vosloo TG, Swarts JC, 1998; β-Diketones containing a ferrocenyl group: synthesis, structural aspects, pK*_{*a*}^{*1*} *values, group electronegativities and complexation with rhodium(I)*. ***Dalton Trans*.,** *2507-2514)*. The published route to the complexes involves at least five intermediates. The Applicant has also found that none of these intermediates, which include ferrocene itself, acetyl ferrocene, esters of the type R⁴COOMe and R⁴COOEt (R⁴ = Fc, CF₃, CCl₃, CH₃, H, Ph or Fc), RhCl₃ and [RhCl(cod)]₂, exhibit any significant anticancer activity. The Applicant has found that the compounds of the invention show better chemotherapeutic and radiosensitisation properties than cisplatin.

### APPENDIX

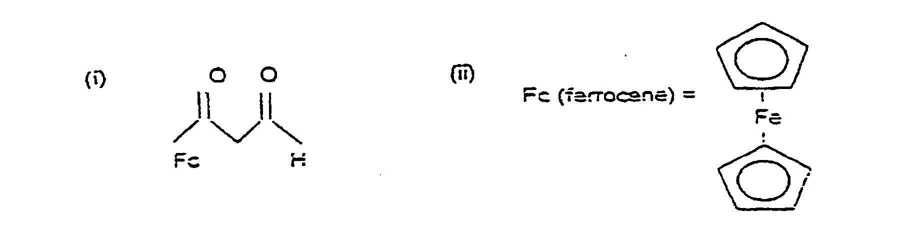

1. (i) The molecular structure of Ferrocenoylacetaidehyde, (Hfch). (ii) Fc refers to ferrocenyl = FeC₁₀H₉ = Fe(C₅H₅)(C₅H₄), a dicyclopentadienyl moiety. 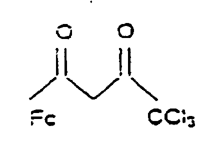
2. The molecular structure of Ferrocenoyltrichloroacetone, (Hfctca). (ii) The molecular structure of cyclooctadiene (cod). 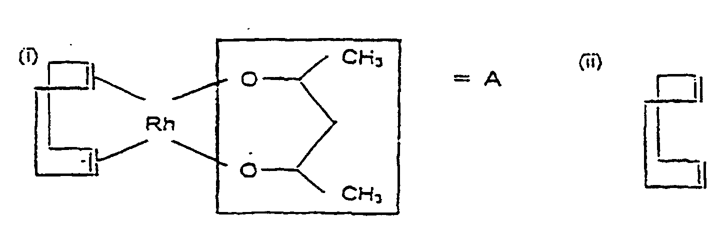 A = acetylacetonato or 1,3-pentanedionato or acac
3. (i) The molecular structure of (η⁴-1,5-cyclooctadiene)(1,3-pentanedionato-κ²O,O')rhodium(1) [Rh(acac)(cod)]. (ii) The molecular structure of cyclooctadiene (cod). 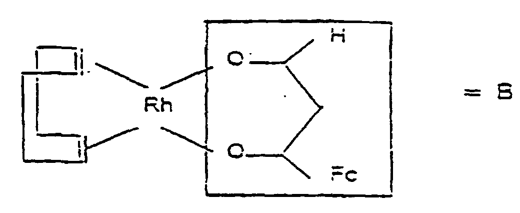 B = 1-ferrocenyl-1,3-propanedionato or FcH
4. The molecular structure of (η⁴-1,5-cyclooctadiene)(1-ferrocenyl-1,3-propanedionato-κ²O,O')rhodium(1) [Rh(FcH)(cod)]. 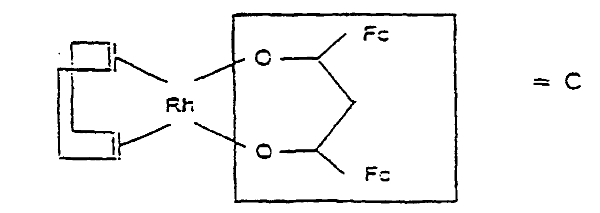 C = 1,3-diferroncenyl-1,3-propanedianato or dfcm
5. The molecular structure of (η⁴-1,5-cyclooctadiene)(1,3-diferrocenyl-1,3-propanedionato-κ²O,O')rhodium(1) [Rh(dfcm)(cod)]. 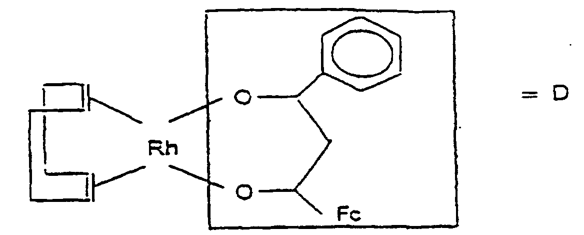 D = 1-ferrocenyl-3-phenyl-1,3-propanedionato or bfcm
6. The molecular structure of (η⁴-1,5-cyclooctadiene)(1-ferrocenyl-3-phenyl-1,3-propanedionato-κ²O,O')rhodium(1) [Rh(bfcm)(cod)]. 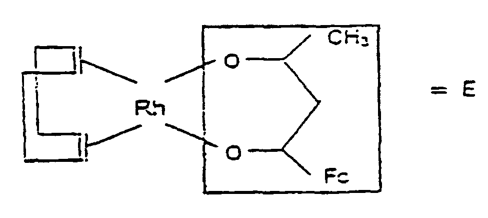 E = ferrocenyolyacetonato or 1-ferrocenyl-1,3-butanedionato or fca
7. The molecular structure of (η⁴-1,5-cyclooctadiene)(1-ferrocenyl-1,3-butanedionato-κ²O,O')rhodium(1) [Rh(fca)(cod)]. 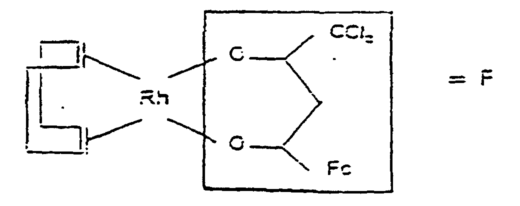 F = ferrocenoyltrichloroacetonato or 1-ferrocenyl-4,4,4-trichloro-1,3-butanedionato or fctca
8. The molecular structure of (η⁴-1,5-cyclooctadiene)(1-ferrocenyl-4,4,4-trichloro-1,3-butanedionato-κ²O,O')rhodium(1) [Rh(fctfa)(cod)]. 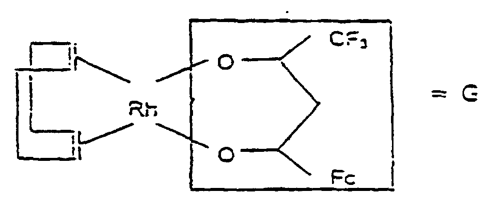 G = ferrocenoyltrifluoroacetonato or 1-ferrocenyl-4,4,4-trifluoro-1,3-butanedionate or fctfa
9. The molecular structure of (η⁴-1,5-cyclooctadiene)(1-ferrocenyl-4,4,4-trifluooro-1,3-butanedionato-κ²O,O')rhodium(1) [Rh(fctfa) (cod)].
10. In all cases Fc in the above structures can be replaced by Rc (ruthenocenyl[Ru(C₅H₅)(C₅H₄)] or Oc (osmocenyl) [Os(C₅H₅)(C₅H₄)].

## Claims

1. Use of a substance or composition in the preparation of a medicament for the chemotherapeutic treatment of cancer, the substance or composition including
(a) at least one compound which is selected from metallocenyl β-diketones of the general formula Mc-CO-CZ₁Z₂-CO-R in which Mc is selected from Fc (ferrocenyl), Rc (ruthenocenyl) and Oc (osmocenyl), R is H, alkyl, haloalkyl, Mc or aryl and Z₁ and Z₂ are independently H, alkyl, aryl or substituted alkyl or ferrocenyl, or
(b) at least one compound which is selected from the enol forms of the β-diketones and metal complexes of the β-diketones of the general formula M(β-diketonato)A¹, M(β-diketonato)A¹A², M(β-diketonato)A¹B¹B², M(β-diketonato)B¹B² and M(β-diketonato)B¹B²B³B⁴ in which
β-diketonato is Mc-CO-CZ₁Z₂-CO-R as described above,
M is selected from Rh and Ir,
A¹ and A² are the same or different and are selected from cyclic dienes having 6 - 8 carbons or linear alkenes having 2 - 7 carbons
B¹, B², B³ and B⁴ are the same or different and are selected from CO, P(R¹R² R³), P(OR¹)(OR²)(OR³), R⁴ and X in which R¹,R², R³ and R⁴ are the same or different and are independently selected from alkyl, phenyl and ferrocenyl, and X is a halide or a pseudohalide.

2. Use as claimed in Claim 1, in which Z₁ and Z₂ are selected from haloalkyl and benzyl.

3. Use as claimed in Claim 1 or Claim 2, in which R is selected from CF₃, CCl₃, CH₃, H, Ph (phenyl) and Mc.

4. Use as claimed in any one of Claims 1 to 3 inclusive, in which the halide is selected from F, Cl, Br and I.

5. Use as claimed in any one of Claims 1 to 4 inclusive, in which the pseudohalide is selected from N₃, NCO and SCN.

6. Use as claimed in any one of Claims 1 to 5 inclusive, in which the compound is selected from the group consisting of [M(β-diketonato)(cod)], [M(β-diketonato)(CO)₂], [M(β-diketonato)(CO)(PR²₃)], [M(β-diketonato){P-(OR²)₃}₂] [M(β-diketonato)(CO)₂(R³)(X)] or its acyl isomer, [M(β-diketonato)(CO)(COR³)(X)], [M(β-diketonato)(CO)(PR²₃)(R³)(X)] or its acyl isomer, [M(β-diketonato)(PR²₃)(COR³)(X)], [M(β-diketonato){P-(OR²)₃}₂(R³)(X)] and [M(β-diketonato)(cod)(R³)(X)], in which M is Rh or Ir, cod is 1,5-cyclooctadiene, (β-diketonato) is (McCOCHCOR) in which R is selected from CF₃, CCl₃, CH₃, H, phenyl and Mc, R² is alkyl, phenyl, ferrocenyl and combinations thereof, R³ is alkyl, phenyl or ferrocenyl , and X is a halide or pseudohalide.

7. Use as claimed in any one of Claims 1 to 6 inclusive, in which the compound is selected from the following compounds 1 - 38:
(1) ferrocenoylacetaldehyde, (Hfch),
(2) (η⁴-1,5-cyclooctadiene)(1-ferroceny)-1,3-propanedionato-κ²O,O')rhodium(1), [Rh(FcH)(cod)],
(3) (η⁴-1,5-cyclooctadiene)(1-ferrocenyl-4,4,4-trifluoro-1,3-butanedionato-κ²O,O')iridium(1), [Ir(fctfa)(cod)],
(4) (η⁴-1,5-cyclooctadiene)(1-ferrocenyl-1,3-butanedionato-κ²O,O')iridium(1), [Ir(fca)(cod)],
(5) (η⁴-1,5-cyclooctadiene)(1-ferrocenyl-3-phenyl-1,3-propanedionato-κ²O,O')iridium(1), [Ir(bfcm)(cod)],
(6) (η⁴-1,5-cyclooctadiene)(1-ferrocenyl-1,3-propanedionato-κ²O,O')iridium(1), [Ir(fch)(cod)].
(7) (η⁴-1,5-cyclooctadiene)(1-ruthenocenyl-1,3-propanedionato-κ²O,O')rhodium(1), [Rh(RcH)(cod)],
(8) (η⁴-1,5-cyclooctadiene)(1,3-diruthenocenyl-1,3-propanedionato-κ²O,O')rhodium(1) [Rh(drcm)(cod)],
(9) (η⁴-1,5-cyclooctadiene)(1-ruthenocenyl-3-phenyl-1,3-propanedionato-κ²O,O')rhodium(1) [Rh(brcm)(cod)],
(10) (η⁴-1,5-cyclooctadiene)(1-ruthenocenyl-1,3-butanedionato-κ²O,O')rhodium(1), [Rh(rca)(cod)],
(11)(η⁴-1,5-cyclooctadiene)(1-ruthenocenyl-4,4,4-trichloro-1,3-butanedionato-κ²O,O')rhodium(1), [Rh(rctca)(cod)],
(12) (η⁴-1,5-cyclooctadiene)(1-ruthenocenyl-4,4,4-trifluooro-1,3-butanedionato-κ²O,O')rhodium(1), [Rh(rctfa)(cod)],
(13)(η⁴-1,5-cyclooctadiene)(1-osmocenyl-1,3-propanedionato-κ²O,O')rhodium(1), [Rh(OcH)(cod)],
(14) (η⁴-1,5-cyclooctadiene)(1,3-diosmocenyl-1,3-propanedionato-κ²O,O')rhodium(1) [Rh(docm)(cod)],
(15) (η⁴-1,5-cyclooctadiene)(1-osmocenyl-3-phenyl-1,3-propanedionato-κ²O,O')rhodium(1) [Rh(bocm)(cod)],
(16) (η⁴-1,5-cyclooctadiene)(1-osmocenyl-1,3-butanedionato-κ²O,O')rhodium(1), [Rh(oca)(cod)],
(17) (η⁴-1,5-cyclooctadiene)(1-osmocenyl-4,4,4-trichloro-1,3-butanedionato-κ²O,O')rhodium(1), [Rh(octca)(cod)],
(18) (η⁴-1,5-cyclooctadiene)(1-osmocenyl-4,4,4-trifluooro-1,3-butanedionato-κ²O,O')rhodium(1), [Rh(octfa)(cod)],
(19) (η⁴-1,5-cyclooctadiene)(1-ferrocenyl-3-ruthenocenyl-1,3-propanedionato-κ²O,O')rhodium(1) [Rh(fcrcm)(cod)],
(20) (η⁴-1,5-cyclooctadiene)(1-ferrocenyl-3-osmocenyl-1,3-propanedionato-κ²O,O')rhodium(1) [Rh(fcocm)(cod)],
(21) (η⁴-1,5-cyclooctadiene)(1-osmocenyl-3-ruthenocenyl-1,3-propanedionato-κ²O,O')rhodium(1) [Rh(ocrcm)(cod)],
(22) 1-ruthenocenyl-1,3-propanedione = HRcH,
(23) 1,3-diruthenocenyl-1,3-propanedione = Hdrcm,
(24) 1-ruthenocenyl-3-phenyl-1,3-propanedione = Hbrcm,
(25) ruthenocenoylacetone = 1-ruthenocenyl-1,3-butanedione = Hrca,
(26) ruthenocenoyltrichloroacetone = 1-ruthenocenyl-4,4,4-trichloro-1,3-butanedione = Hrctca,
(27) ruthenocenoyltrifluoroacetone = 1-ruthenocenyl-4,4,4-trifluoro-1,3-butanedione = Hrctfa,
(28) 1-osmocenyl-1,3-propanedione = HOcH
(29) 1,3-diosmocenyl-1,3-propanedione = Hdocm
(30) 1-osmocenyl-3-phenyl-1,3-propanedione = Hbocm,
(31) osmocenoylacetone = 1-osmocenyl-1,3-butanedione = Hoca,
(32) osmocenoyltrichloroacetone = 1-osmocenyl-4,4,4-trichloro-1,3-butanedione = Hoctca,
(33)osmocenoyltrifluoroacetone = 1-osmocenyl-4,4,4-trifluoro-1,3-butanedione = Hoctfa,
(34) ferrocenoylosmocenoylmethane = 1 ferrocenyl-3-osmocenyl-1,3-propanedione = Hfcocm,
(35) osmocenoylruthenocenoylmethane = 1-osmocenyl-3-ruthenocenyl-1,3-propanedione = Hrcocm,
(36) ferrocenoylruthertocenoylmethane = 1-ferrocenyl-3-ruthenocenyl-1,3-propanedione = Hfcrcm,
(37) (η⁴-1,5-cyclooctadiene)(1-ferrocenyl-3-ruthenocenyl-1,3-propanedionato-κ²O,O')rhodium(1) [Rh(fcrcm)(cod)],
(38) (η⁴-1,5-cyclooctadiene)(1-ferrocenyl-3-osmocenyl-1,3-propanedionato-κ²O,O')rhodium(1) [Rh(fcocm)(cod)]

8. Use of a substance or composition for the preparation of a medicament for use in sensitizing cells to radiation, the substance or composition including
(a) at least one compound which is selected from metallocenyl β-diketones of the general formula Mc-CO-CZ₁Z₂-CO-R in which Mc is selected from Fc (ferrocenyl), Rc (ruthenocenyl) and Oc (osmocenyl), R is H, alkyl, haloalkyl, Mc or aryl and Z₁ and Z₂ are independently H, alkyl, aryl or substituted alkyl or ferrocenyl, or
(b) at least one compound which is selected from the enol forms of the β-diketones and metal complexes of the β-diketones of the general formula M(β-diketonato)A¹, M(β-diketonato)A¹A², M(β-diketonato)A¹B¹B², M(β-diketonato)B¹B² and M(β-diketonato)B¹B²B³B⁴ in which
β-diketonato is Mc-CO-CZ₁Z₂-CO-R as described above,
M is selected from Rh and Ir,
A¹ and A² are the same or different and are selected from cyclic dienes having 6 - 8 carbons or linear alkenes having 2 - 7 carbons
B¹, B², B³ and B⁴ are the same or different and are selected from CO, P(R¹R² R³), P(OR¹)(OR²)(OR³), R⁴ and X in which R¹,R², R³ and R⁴ are the same or different and are independently selected from alkyl, phenyl and ferrocenyl, and X is a halide or a pseudohalide.

9. Use as claimed in Claim 8, in which Z₁ and Z₂ are selected from haloalkyl and benzyl.

10. Use as claimed in Claim 8 or Claim 9, in which R is selected from CF₃, CCl₃, CH₃, H, Ph (phenyl) and Mc.

11. Use as claimed in any one of Claims 8 to 10 inclusive, in which the halide is selected from F, Cl, Br and I.

12. Use as claimed in any one of Claims 8 to 11 inclusive, in which the pseudohalide is selected from N₃, NCO and SCN.

13. Use as claimed in any one of Claims 8 to 12 inclusive, in which the compound is selected from the group consisting of [M(β-diketonato)(cod)], [M(β-diketonato)(CO)₂], (M(β-diketonato)(CO)(PR²₃)], [M(β-diketonato){P-(OR²)₃}₂] [M(β-diketonato)(CO)₂(R³)(X)] or its acyl isomer, [M(β-diketonato)(CO)(COR³)(X)], [M(β-diketonato)(CO)(PR²₃)(R³)(X)] or its acyl isomer, [M(β-diketonato)(PR²₃)(COR³)(X)], [M(β-diketonato){P-(OR²)₃}₂(R³)(X)] and [M(β-diketonato)(cod)(R³)(X)], in which M is Rh or Ir, cod is 1,5-cyclooctadiene, (β-diketonato) is (McCOCHCOR) in which R is selected from CF₃, CCl₃, CH₃, H, phenyl and Mc, R² is alkyl, phenyl, ferrocenyl and combinations thereof, R³ is alkyl, phenyl or ferrocenyl , and X is a halide or pseudohalide.

14. Use as claimed in any one of Claims 8 to 13 inclusive, in which the compound is selected from the following compounds 1 - 48
(1) ferrocenoylacetaldehyde, (Hfch),
(2) ferrocenoyltrichloroacetone, (Hfctca),
(3) (η⁴-1,5-cyclooctadiene)(1-ferrocenyl-1,3-propanedionato-κ²O,O')rhodium(1), [Rh(FcH)(cod)],
(4) (η⁴-1,5-cyclooctadiene)(1,3-diferrocenyl-1,3-propanedionato-κ²O,O')rhodium(1) [Rh(dfcm)(cod)],
(5) (η⁴-1,5-cyclooctadiene)(1-ferrocenyl-3-phenyl-1,3-propanedionato-κ²O,O')rhodium(1) [Rh(bfcm)(cod)],
(6) (η⁴-1,5-cyclooctadiene)(1-ferrocenyl-1,3-butanedionato-κ²O,O')rhodium(1), [Rh(fca)(cod)],
(7) (η⁴-1,5-cyclooctadiene)(1-ferrocenyl-4,4,4-trichloro-1,3-butanedionato-κ²O,O')rhodium(1), [Rh(fctca)(cod)],
(8) (η⁴-1,5-cyclooctadiene)(1-ferrocenyl-4,4,4-trifluooro-1,3-butanedionato-κ²O,O')rhodium(1), [Rh(fctfa)(cod)],
(9) diferrocenoylmethane, (Hdfcm),
(10) ferrocenoyltrifluoroacetone, (Hfctfa),
(11) (η⁴-1,5-cyclooctadiene)(1-ferrocenyl-4,4,4-trifluoro-1,3-butanedionato-κ²O,O')iridium(1), [Ir(fctfa)(cod)],
(12) (η⁴-1,5-cyclooctadiene)(1-ferrocenyl-1,3-butanedionato-κ²O,O')iridium(1), [Ir(fca)(cod)],
(13) (η⁴-1,5-cyclooctadiene)(1-ferrocenyl-3-phenyl-1,3-propanedionato-κ²O,O')iridium(1), [Ir(bfcm)(cod)],
(14) benzoylferrocenoylmethane, (Hbfcm),
(15) ferrocenoylacetone, (Hfca),
(16) (η⁴-1,5-cyclooctadiene)(1-ferrocenyl-1,3-propanedionato-κ²O,O')iridium(1), [Ir(fch)(cad)],
(17) (η⁴-1,5-cyclooctadiene)(1-ruthenocenyl-1,3-propanedionato-κ²O,O')rhodium(1), [Rh(RcH)(cod)],
(18) (η⁴-1,5-cyclooctadiene)(1,3-diruthenocenyl-1,3-propanedionato-κ²O,O')rhodium(1) [Rh(drcm)(cod)],
(19) (η⁴-1,5-cyclooctadiene)(1-ruthenocenyl-3-phenyl-1,3-propanedionato-κ²O,O')rhodium(1) [Rh(brcm)(cod)],
(20) (η⁴-1,5-cyclooctadiene)(1-ruthenocenyl-1,3-butanedionato-κ²O,O')rhodium(1), [Rh(rca)(cod)],
(21) (η⁴-1,5-cyclooctadiene)(1-ruthenocenyl-4,4,4-trichloro-1,3-butanedionato-κ²O,O')rhodium(1), [Rh(rctca)(cod)],
(22) (η⁴-1,5-cyclooctadiene)(1-ruthenoceny)-4,4,4-trifluooro-1,3-butanedionato-κ²O,O')rhodium(1), [Rh(rctfa)(cod)],
(23) (η⁴-1,5-cyclooctadiene)(1-osmocenyl-1,3-propanedionato-κ²O,O')rhodium(1), [Rh(OcH)(cod)],
(24) (η⁴-1,5-cyclooctadiene)(1,3-diosmocenyl-1,3-propanedionato-κ²O,O')rhodium(1) [Rh(docm)(cod)],
(25) (η⁴-1,5-cyclooctadiene)(1-osmocenyl-3-phenyl-1,3-propanedionato-κ²O,O')rhodium(1) [Rh(bocm)(cod)],
(26) (η⁴-1,5-cyclooctadiene)(1-osmocenyl-1,3-butanedionato-κ²O,O')rhodium(1), [Rh(oca)(cod)],
(27) (η⁴-1,5-cyclooctadiene)(1-osmocenyl-4,4,4-trichloro-1,3-butanedionato-κ²O,O')rhodium(1), [Rh(octca)(cod)],
(28) (η⁴-1,5-cyclooctadiene)(1-osmocenyl-4,4,4-trifluooro-1,3-butanedionato-κ²O,O')rhodium(1), [Rh(octfa)(cod)],
(29) (η⁴-1,5-cyclooctadiene)(1-ferrocenyl-3-ruthenocenyl-1,3-propanedionato-κ²O,O')rhodium(1) [Rh(fcrcm)(cod)],
(30) (η⁴-1,5-cyclooctadiene)(1-ferrocenyl-3-osmocenyl-1,3-propanedionato-κ²O,O')rhodium(1) [Rh(fcocm)(cod)],
(31) (η⁴-1,5-cyclooctadiene)(1-osmocenyl-3-ruthenocenyl-1,3-propanedionato-κ²O,O')rhodium(1) [Rh(ocrcm)(cod)],
(32) 1-ruthenocenyl-1,3-propanedione = HRcH,
(33) 1,3-diruthenocenyl-1,3-propanedione = Hdrcm,
(34) 1-ruthenocenyl-3-phenyl-1,3-propanedione = Hbrcm,
(35) ruthenocenoylacetone = 1-ruthenocenyl-1,3-butanedione = Hrca,
(36) ruthenocenoyltrichloroacetone = 1-ruthenocenyl-4,4,4-trichloro-1,3-butanedione = Hrctca,
(37) ruthenocenoyltrifluoroacetone = 1-ruthenocenyl-4,4,4-trifluoro-1,3-butanedione = Hrctfa,
(38) 1-osmocenyl-1,3-propanedione = HOcH
(39) 1,3-diosmocenyl-1,3-propanedione = Hdocm
(40) 1-osmocenyl-3-phenyl-1,3-propanedione = Hbocm,
(41) osmocenoylacetone = 1-osmocenyl-1,3-butanedione = Hoca,
(42) osmocenoyltrichloroacetone = 1-osmocenyl-4,4,4-trichloro-1,3-butanedione = Hoctca,
(43) osmocenoyltrifluoroacetone = 1-osmocenyl-4,4,4-trifluoro-1,3-butanedione = Hoctfa,
(44) ferrocenoylosmocenoylmethane = 1 ferrocenyl-3-osmocenyl-1,3-propanedione = Hfcocm,
(45) osmocenoylruthenocenoylmethane = 1-osmocenyl-3-ruthenocenyl-1,3-propanedione = Hrcocm,
(46) ferrocenoylruthenocenoylmethane = 1-ferrocenyl-3-ruthenocenyl-1,3-propanedione = Hfcrcm,
(47) (η⁴-1,5-cyclooctadiene)(1-ferrocenyl-3-ruthenocenyl-1,3-propanedionato-κ²O,O')rhodium(1) [Rh(fcrcm)(cod)],
(48) (η⁴-1,5-cyclooctadiene)(1-ferrocenyl-3-osmocenyl-1,3-propanedionato-κ²O,O')rhodium(1) [Rh(fcocm)(cod)]

15. A compound which is:
(a) selected from metallocenyl β-diketones of the general formula Mc-CO-CZ₁Z₂-CO-R in which Mc is selected from Fc (ferrocenyl), Rc (ruthenocenyl) and Oc (osmocenyl), R is H, alkyl, haloalkyl, Mc or aryl and Z₁ and Z₂ are independently H, alkyl aryl or substituted alkyl or ferrocenyl, or
(b) selected from the enol forms of the β-diketones and metal complexes of the β-diketones of the general formula M(β-diketonato)A¹, M(β-diketonato)A¹A², M(β-diketonato)A¹B¹B², M(β-diketonato)B¹B² and M(β-diketonato)B¹B²B³B⁴ in which
β-diketonato is Mc-CO-CZ₁Z₂-CO-R as described above,
M is selected from Rh and Ir,
A¹ and A² are the same or different and are selected from cyclic dienes having 6 - 8 carbons, or linear alkenes having 2-7 carbons
B¹, B², B³ and B⁴ are the same or different and are selected from CO, P(R¹R²R³), P(OR¹)(OR²)(OR³), R⁴ and X in which R¹,R², R³ and R⁴ are the same or different and are independently selected from alkyl, phenyl and ferrocenyl, and X is a halide or a pseudohalide, with the proviso that the compound may not be
(1) ferrocenoyltrichloroacetone, (Hfctca),
(2) (η⁴-1,5-cyclooctadiene)(1,3-diferrocenyl-1,3-propanedionato-κ²O,O')rhodium(1) [Rh(dfcm)(cod)],
(3) (η⁴-1,5-cyclooctadiene)(1-ferrocenyl-3-phenyl-1,3-propanedionato-κ²O,O')rhodium(1) [Rh(bfcm)(cod)],
(4) (η⁴-1,5-cyclooctadiene)(1-ferrocenyl-1,3-butanedionato-κ²O,O')rhodium(1), [Rh(fca)(cod)],
(5) (η⁴-1,5-cyclooctadiene)(1-ferrocenyl-4,4,4-trichloro-1,3-butanedionato-κ²O,O')rhodium(1), [Rh(fctca)(cod)],
(6) (η⁴-1,5-cyclooctadiene)(1-ferrocenyl-4,4,4-trifluooro-1,3-butanedionato-κ²O,O')rhodium(1), [Rh(fctfa)(cod)],
(7) diferrocenoylmethane, (Hdfcm),
(8) ferrocenoyltrifluoroacetone, (Hfctfa),
(9) benzoylferrocenoylmethane, (Hbfcm),
(10) ferrocenoylacetone, (Hfca),
(11) (η⁴-1,5-cyclooctadiene)(1,3-pentanedionato-κ²O,O')rhodium(1) [Rh(acac)(cod)],
(12) (η²-1,2-ethene)₂(1-ferrocenyl-1,3-butanedionato-k²O,O')rhodium(1),[Rh(fca)(CH₂CH₂)₂]
(13) ferrocenoylacetaldehyde, (Hfch)

16. A compound as claimed in Claim 15, in which Z₁ and Z₂ are selected from haloalkyl and benzyl.

17. A compound as claimed in Claim 1 5 or Claim 16, in which R is selected from CF₃, CCl₃, CH₃, H, Ph (phenyl) and Mc.

18. A compound as claimed in any one of Claims 15 to 17 inclusive, in which the halide is F, Cl, Br or I.

19. A compound as claimed in any one of Claims 15 to 18 inclusive, in which the pseudohalide is N₃, NCO or SCN.

20. A compound as claimed in any one of Claims 15 to 19 inclusive, which is selected from the group consisting of [M(β-diketonato)(cod)], [M(β-diketonato)(CO)₂], [M(β-diketonato)(CO)(PR²₃)], [M(β-diketonato){P-(OR²)₃}₂] [M(β-diketonato)(CO)₂(R³)(X)] or its acyl isomer [M(β-diketonato)(CO)(COR³)(X)], [M(β-diketonato)(CO)(PR²₃)(R³)(X)] or its acyl isomer [M(β-diketonato)(PR²₃)(COR³](X)], [M(β-diketonato){P-(OR²)₃}₂(R³)(X)] and [M(β-diketonato)(cod)(R³)(X)], in which M is Rh or Ir, cod is 1,5-cyclooctadiene, (β-diketonato) is (McCOCHCOR) in which R is selected from CF₃, CCl₃, CH₃, H, phenyl and Mc, R² is alkyl, phenyl, ferrocenyl and combinations thereof, R³ is alkyl, phenyl or ferrocenyl, and X is a halide or pseudohalide.

21. A compound as claimed in any one of Claims 15 to 20 inclusive, which is selected from the group consisting of the following compounds 1 - 37
(1) (η⁴-1,5-cyclooctadiene)(1-ferrocenyl-1,3-propanedionato-κ²O,O')rhodium(1), [Rh(FcH)(cod)],
(2) (η⁴-1,5-cyclooctadiene)(1-ferrocenyl-4,4,4-trifluoro-1,3-butanedionato-κ²O,O')iridium(1), [Ir(fctfa)(cod)],
(3) (η⁴-1,5-cyclooctadiene)(1-ferrocenyl-1,3-butanedionato-κ²O,O')iridium(1), [Ir(fca)(cod)],
(4) (η⁴-1,5-cyclooctadiene)(1-ferrocenyl-3-phenyl-1,3-propanedionato-κ²O,O')iridium(1), [Ir(bfcm)(cod)],
(5) (η⁴-1,5-cyclooctadiene)(1-ferrocenyl-1,3-propanedionato-κ²O,O')iridium(1), [Ir(fch)(cod)].
(6) (η⁴-1,5-cyclooctadiene)(1-ruthenocenyl-1,3-propanedionato-κ²O,O')rhodium(1), [Rh(RcH)(cod)],
(7) (η⁴-1,5-cyclooctadiene)(1,3-diruthenocenyl-1,3-propanedionato-κ²O,O')rhodium(1) [Rh(drcm)(cod)],
(8) (η⁴-1,5-cyclooctadiene)(1-ruthenocenyl-3-phenyl-1,3-propanedionato-κ²O,O')rhodium(1) [Rh(brcm)(cod)],
(9) (η⁴-1,5-cyclooctadiene)(1-ruthenocenyl-1,3-butanedionato-κ²O,O')rhodium(1), [Rh(rca)(cod)],
(10) (η⁴-1,5-cyclooctadiene)(1-ruthenocenyl-4,4,4-trichloro-1,3-butanedionato-κ²O,O')rhodium(1), [Rh(rctca)(cod)],
(11) (η⁴-1,5-cyclooctadiene)(1-ruthenocenyl-4,4,4-trifluooro-1,3-butanedionato-κ²O,O')rhodium(1), [Rh(rctfa)(cod)],
(12) (η⁴-1,5-cyclooctadiene)(1-osmocenyl-1,3-propanedionato-κ²O,O')rhodium(1), [Rh(OcH)(cod)],
(13) (η⁴-1,5-cyclooctadiene)(1,3-diosmocenyl-1,3-propanedionato-κ²O,O')rhodium(1) [Rh(docm)(cod)],
(14) (η⁴-1,5-cyclooctadiene)(1-osmocenyl-3-phenyl-1,3-propanedionato-κ²O,O')rhodium(1) [Rh(bocm)(cod)],
(15) (η⁴-1,5-cyclooctadiene)(1-osmocenyl-1,3-butanedionato-κ²O,O')rhodium(1), [Rh(oca)(cod)],
(16) (η⁴-1,5-cyclooctadiene)(1-osmocenyl-4,4,4-trichloro-1,3-butanedionato-κ²O,O')rhodium(1), [Rh(octca)(cod)],
(17) (η⁴-1,5-cyclooctadiene)(1-osmocenyl-4,4,4-trifluooro-1,3-butanedionato-κ²O,O')rhodium(1), [Rh(octfa)(cod)],
(18) (η⁴-1,5-cyclooctadiene)(1-ferrocenyl-3-ruthenocenyl-1,3-propanedionato-κ²O,O')rhodium(1) [Rh(fcrcm)(cod)],
(19) (η⁴-1,5-cyclooctadiene)(1-ferrocenyl-3-osmocenyl-1,3-propanedionato-κ²O,O')rhodium(1) [Rh(fcocm)(cod)],
(20) (η⁴-1,5-cyclooctadiene)(1-osmocenyl-3-ruthenocenyl-1,3-propanedionato-κ²O,O')rhodium(1) [Rh(ocrcm)(cod)],
(21) 1-ruthenocenyl-1,3-propanedione = HRcH,
(22) 1,3-diruthenocenyl-1,3-propanedione = Hdrcm,
(23) 1-ruthenocenyl-3-phenyl-1,3-propanedione = Hbrcm,
(24) ruthenocenoylacetone = 1-ruthenocenyl-1,3-butanedione = Hrca,
(25) ruthenocenoyltrichloroacetone = 1-ruthenocenyl-4,4,4-trichloro-1,3-butanedione = Hrctca,
(26) ruthenocenoyltrifluoroacetone = 1-ruthenocenyl-4,4,4-trifluoro-1,3-butanedione = Hrctfa,
(27) 1-osmocenyl-1,3-propanedione = HOcH
(28) 1,3-diosmocenyl -1,3-propanedione = Hdocm
(29) 1-osmocenyl-3-phenyl-1,3-propanedione = Hbocm,
(30) osmocenoylacetone = 1-osmocenyl-1,3-butanedione = Hoca,
(31) osmocenoyltrichloroacetone = 1-osmocenyl-4,4,4-trichloro-1,3-butanedione = Hoctca,
(32)osmocenoyltrifluoroacetone = 1-osmocenyl-4,4,4-trifluoro-1,3-butanedione = Hoctfa,
(33) ferrocenoylosmocenoylmethane = 1 ferrocenyl-3-osmocenyl-1,3-propanedione = Hfcocm,
(34) osmocenoylruthenocenoylmethane = 1-osmocenyl-3-ruthenocenyl-1,3-propanedione = Hrcocm,
(35) ferrocenoylruthenocenoylmethane = 1-ferrocenyl-3-ruthenocenyl-1,3-propanedione = Hfcrcm,
(36) (η⁴-1,5-cyclooctadiene)(1-ferrocenyl-3-ruthenocenyl-1,3-propanedionato-κ²O,O')rhodium(1) [Rh(fcrcm)(cod)],
(37) (η⁴-1,5-cyclooctadiene)(1-ferrocenyl-3-osmocenyl-1,3-propanedionato-κ²O,O')rhodium(1) [Rh(fcocm)(cod)]

## Patentansprüche

1. Verwendung einer Substanz oder Zusammensetzung bei der Herstellung eines Medikaments für die chemotherapeutische Behandlung von Krebs,
wobei die Substanz oder Zusammensetzung enthält
(a) wenigstens eine Verbindung, welche ausgewählt wird aus Metallocenyl β-Diketonen der allgemeinen Formel Mc-CO-CZ₁Z₂-CO-R, in welcher Mc ausgewählt wird aus Fc (Ferrocenyl), Rc (Ruthenocenyl) und Oc (Osmocenyl), R ist H, Alkyl, Haloalkyl, Mc oder Aryl und Z₁ und Z₂ unabhängig voneinander H, Alkyl, Aryl oder ein substituiertes Alkyl oder Ferrocenyl sind, oder
(b) wenigstens eine Verbindung, welche ausgewählt wird aus Enolformen der β-Diketone und der Metallkomplexe der β-Diketone der allgemeinen Formel M(β-Diketonato)A¹, M(β-Diketonato)A¹A², M(β-Diketonato)A¹B¹B², M(β-Diketonato)B¹B² und M(β-Diketonato) B¹B²B³B⁴, in welchen
β-Diketonato ein Mc-CO-CZ₁Z₂-CO-R ist, wie dies oben beschrieben wurde,
M ausgewählt ist aus Rh und Ir,
A¹ und A² gleich oder unterschiedlich sind und ausgewählt werden aus zyklischen Dienen mit 6 - 8 Kohlenstoffen oder linearen Alkenen mit 2 - 7 Kohlenstoffen
B¹B², B³ und B⁴ gleich oder unterschiedlich sind und ausgewählt werden aus CO, P(R¹R²R³), P(OR¹)(OR²)(OR³),
R⁴ und X, in welchen R¹, R², R³ und R⁴ gleich oder unterschiedlich sind und unabhängig voneinander ausgewählt werden aus Alkyl, Phenyl und Ferrocenyl und X ein Halid oder ein Pseudohalid ist.

2. Verwendung nach Anspruch 1, in welcher Z₁ und Z₂ ausgewählt wird aus Haloalkyl und Benzyl.

3. Verwendung nach Anspruch 1 oder Anspruch 2, in welcher R ausgewählt wird aus CF₃, CCl₃, CH₃, H, Ph (Phenyl) und Mc.

4. Verwendung nach einem der Ansprüche 1 bis 3 einschließlich, in welcher das Halid ausgewählt wird aus F, Cl, Br und J.

5. Verwendung nach einem der Ansprüche 1 bis 4 einschließlich, in welcher das Pseudohalid ausgewählt wird aus N₃, NCO und SCN.

6. Verwendung nach einem der Ansprüche 1 bis 5 einschließlich, in welcher die Verbindung ausgewählt wird aus der Gruppe bestehend aus [M(β-Diketonato)(cod)], [M(β-Diketonato)(CO)₂], [M(β-Diketonato)(CO)(PR²₃)], [M(β-Diketonato){P-(OR²)₃}₂] [M(β-Diketonato)(CO)₂(R³)(X)] oder seines Acylisomers, [M(β-Diketonato)(CO)(COR³)(X)], [M(β-Diketonato)(CO)(PR²₃)(R³)(X)] oder seines Acylisomers, [M(β-Diketonato)(PR²₃)(COR³)(X)], [M(β-Diketonato){P-(OR²)₃}₂(R³)(X)] und [M(β-Diketonato)(cod)R³)(X)], in welcher M ist Rh oder Ir, cod ist 1,5-cyclooctadien, (β-Diketonato) ist (McCOCHCOR), in welchen R ausgewählt wird aus CF₃, CCl₃, CH₃, H, Phenyl und Mc, R² ist Alkyl, Phenyl, Ferrocenyl und Kombinationen davon, R³ ist Alkyl, Phenyl oder Ferrocenyl und X ein Halid oder Pseudohalid ist.

7. Verwendung nach einem der Ansprüche 1 bis 6 einschließlich, in welcher die Verbindung ausgewählt wird aus den folgenden Verbindungen 1 - 38:
(1) Ferrocenoylacetaldehyd, (Hfch),
(2) (η⁴-1,5-cyclooctadien)(1-ferrocenyl-1,3-propandionato-(4) κ²O,O')Rhodium(1), [Rh(FcH)(cod)],
(3) (η⁴-1,5-cyclooctadien)(1-ferrocenyl-4,4,4-trifluoro-1,3-butandionato-κ²O,O')Iridium(1), [Ir(fctfa)(cod)],
(4) (η⁴-1,5-cyclooctadien)(1-ferrocenyl-1,3-butandionato-κ²O,O')Iridium(1), [Ir(fca)(cod)],
(5) (η⁴-1,5-cyclooctadien)(1-ferrocenyl-3-phenyl-1,3-propandionato-κ²O,O')Iridium(1), [Ir(bfcm)(cod)],
(6) (η⁴-1,5-cyclooctadien)(1-ferrocenyl-1,3-propandionato-κ²O,O')Iridium(1), [Ir(fch)(cod)].
(7) (η⁴-1,5-cyclooctadien)(1-ruthenocenyl-1,3-propandionato-κ²O,O')Rhodium(1), [Rh(RcH)(cod)],
(8) (η⁴-1,5-cyclooctadien)(1,3-diruthenocenyl-1,3-propandionato-κ²O,O')Rhodium(1) [Rh(drcm)(cod)],
(9) (η⁴-1,5-cyclooctadien)(1-ruthenocenyl-3-phenyl-1,3-propandionato-κ²O,O')Rhodium(1) [Rh(brcm)(cod)],
(10) (η⁴-1,5-cyclooctadien)(1-ruthenocenyl-1,3-butandionato-κ²O,O')Rhodium(1), [Rh(rca)(cod)],
(11) (η⁴-1,5-cyclooctadien)(1-ruthenocenyl-4,4,4-trichloro-1,3-butandionato-κ²O,O')Rhodium(1), [Rh(rctca)(cod)],
(12) (η⁴-1,5-cyclooctadien)(1-ruthenocenyl-4,4,4-trifluoro-1,3-butandionato-κ²O,O')Rhodium(1), [Rh(rctfa)(cod)],
(13) (η⁴-1,5-cyclooctadien)(1-osmocenyl-1,3-propandionato-κ²O,O')Rhodium(1), [Rh(OcH)(cod)],
(14) (η⁴-1,5-cyclooctadien)(1,3-diosmocenyl-1,3-propandionato-κ²O,O')Rhodium(1) [Rh(docm)(cod)],
(15) (η⁴-1,5-cyclooctadien)(1-osmocenyl-3-phenyl-1,3-propandionato-κ²O,O')Rhodium(1) [Rh(bocm)(cod)],
(16) (η⁴-1,5-cyclooctadien)(1-osmocenyl-1,3-butandionato-κ²O,O')Rhodium(1), [Rh(oca)(cod)],
(17) (η⁴-1,5-cyclooctadien)(1-osmocenyl-4,4,4-trichloro-1,3-butandionato-κ²O,O')Rhodium(1), [Rh(octca)(cod)],
(18) (η⁴-1,5-cyclooctadien)(1-osmocenyl-4,4,4-trifluooro-1,3-butandionato-κ²O,O')Rhodium(1), [Rh(octfa)(cod)],
(19) (η⁴-1,5-cyclooctadien)(1-ferrocenyl-3-ruthenocenyl-1,3-propandionato-κ²O,O')Rhodium(1) [Rh(fcrcm)(cod)],
(20) (η⁴-1,5-cyclooctadien)(1-ferrocenyl-3-osmocenyl-1,3-propandionato-κ²O,O')Rhodium(1) [Rh(fcocm)(cod)],
(21) (η⁴-1,5-cyclooctadien)(1-osmocenyl-3-ruthenocenyl-1,3-propandionato-κ²O,O')Rhodium(1) [Rh(ocrcm)(cod)],
(22) 1-ruthenocenyl-1,3-propandion = HRcH,
(23) 1,3-diruthenocenyl-1,3-propandion = Hdrcm,
(24) 1-ruthenocenyl-3-phenyl-1,3-propandion = Hbrcm,
(25) Ruthenocenoylaceton = 1-ruthenocenyl-1,3-butandion = Hrca,
(26) Ruthenocenoyltrichloroaceton = 1-ruthenocenyl-4,4,4-trichloro-1,3-butandion = Hrctca,
(27) Ruthenocenoyltrifluoroaceton = 1-ruthenocenyl-4,4,4-trifluoro-1,3-butandion = Hrctfa,
(28) 1-osmocenyl-1,3-propandion = HOcH
(29) 1,3-diosmocenyl-1,3-propandion = Hdocm
(30) 1-osmocenyl-3-phenyl-1,3-propandion = Hbocm,
(31) Osmocenoylaceton = 1-osmocenyl-1,3-butandion = Hoca,
(32) Osmocenoyltrichloroaceton = 1-osmocenyl-4,4,4-trichloro-1,3-butandion = Hoctca,
(33) Osmocenoyltrifluoroaceton = 1-osmocenyl-4,4,4-trifluoro-1,3-butandion = Hoctfa,
(34) Ferrocenoylosmocenoylmethan = 1 ferrocenyl-3-osmocenyl-1,3-propandion = Hfcocm,
(35) Osmocenoylruthenocenoylmethan = 1-osmocenyl-3-ruthenocenyl-1,3-propanedion = Hrcocm,
(36) Ferrocenoylruthenocenoylmethan = 1-ferrocenyl-3-ruthenocenyl-1,3-propanedion = Hfcrcm,
(37) (η⁴-1,5-cyclooctadien)(1-ferrocenyl-3-ruthenocenyl-1,3-propandionato-κ²O,O')Rhodium(1) [Rh(fcrcm)(cod)],
(38) (η⁴-1,5-cyclooctadien)(1-ferrocenyl-3-osmocenyl-1,3-propandionato-κ²O,O')Rhodium(1) [Rh(fcocm)(cod)]

8. Verwendung einer Substanz oder Zusammensetzung für die Herstellung eines Medikaments zur Verwendung bei der Sensibilisierung von Zellen für eine Strahlung, wobei die Substanz oder Zusammensetzung enthält:
(a) wenigstens eine Verbindung, welche ausgewählt wird aus Metallocenyl β-Diketonen der allgemeinen Formel Mc-CO-CZ₁Z₂-CO-R, in welcher Mc ausgewählt wird aus Fc (Ferrocenyl), Rc (Ruthenocenyl) und Oc (Osmocenyl), R ist H, Alkyl, Haloalkyl, Mc oder Aryl und Z₁ und Z₂ unabhängig voneinander H, Alkyl, Aryl oder ein substituiertes Alkyl oder Ferrocenyl, oder
(b) wenigstens eine Verbindung, welche ausgewählt wird aus Enolformen der β-Diketone und der Metallkomplexe der β-Diketone der allgemeinen Formel M(β-Diketonato)A¹, M(β-Diketonato)A¹A², M(β-Diketonato)A¹B¹B², M(β-Diketonato)B¹B² und M(β-Diketonato) B¹B²B³B⁴, in welchen
β-Diketonato ein Mc-CO-CZ₁Z₂-CO-R ist, wie dies oben beschrieben wurde,
M ausgewählt wird aus Rh und Ir,
A¹ und A² gleich oder unterschiedlich sind und ausgewählt werden aus zyklischen Dienen mit 6 - 8 Kohlenstoffen oder linearen Alkenen mit 2 - 7 Kohlenstoffen B¹B², B³ und B⁴ gleich oder unterschiedlich sind und ausgewählt werden aus CO, P(R¹R²R³), P(OR¹)(OR²)(OR³),
R⁴ und X, in welchen R¹, R², R³ und R⁴ gleich oder unterschiedlich sind und unabhängig voneinander ausgewählt werden aus Alkyl, Phenyl und Ferrocenyl und X ein Halid oder ein Pseudohalid ist.

9. Verwendung nach Anspruch 8, in welcher Z₁ und Z₂ ausgewählt werden aus Haloalkyl und Benzyl.

10. Verwendung nach Anspruch 8 oder Anspruch 9, in welcher R ausgewählt wird aus CF₃, CCl₃, CH₃, H, Ph (Phenyl) und Mc.

11. Verwendung nach einem der Ansprüche 8 bis 10 einschließlich, in welcher das Halid ausgewählt wird aus F, Cl, Br und J.

12. Verwendung nach einem der Ansprüche 8 bis 11 einschließlich, in welcher das Pseudohalid ausgewählt wird aus N₃, NCO und SCN.

13. Verwendung nach einem der Ansprüche 8 bis 12 einschließlich, in welcher die Verbindung ausgewählt wird aus der Gruppe bestehend aus [M(β-Diketonato)(cod)], [M(β-Diketonato)(CO)₂], [M(β-Diketonato)(CO)(PR²₃)], [M(β-Diketonato){P-(OR²)₃}₂] [M(β-Diketonato)(CO)₂(R³)(X)] oder seines Acylisomers, [M(β-Diketonato)(CO)(COR³)(X)], [M(β-Diketonato)(CO)(PR²₃)(R³)(X)] oder seines Acylisomers, [M(β-Diketonato)(PR²₃)(COR³)(X)], [M(β-Diketonato){P-(OR²)₃}₂(R³)(X)] und [M(β-Diketonato)(cod)R³)(X)], in welchen M ist Rh oder Ir, cod ist 1,5-cyclooctadien, (β-Diketonato) ist (McCOCHCOR) ist, in welchen R ausgewählt wird aus CF₃, CCl₃, CH₃, H, Phenyl und Mc, R² ist Alkyl, Phenyl, Ferrocenyl und Kombinationen davon, R³ ist Alkyl, Phenyl oder Ferrocenyl und X ein Halid oder Pseudohalid ist.

14. Verwendung nach einem der Ansprüche 8 bis 13 einschließlich, in welcher die Verbindung ausgewählt wird aus den folgenden Verbindungen 1 - 48:
(1) Ferrocenoylacetaldehyd, (Hfch),
(2) Ferrocenoyltrichloroaceton, (Hfctca),
(3) (η⁴-1,5-cyclooctadien)(1-ferrocenyl-1,3-propandionato-(4) κ²O,O')Rhodium(1), [Rh(FcH)(cod)],
(4) (η⁴-1,5-cyclooctadien)(1,3-diferrocenyl-1,3-propandionato-κ²O,O')Rhodium(1) [Rh(dfcm)(cod)],
(5) (η⁴-1,5-cyclooctadien)(1-ferrocenyl-3-phenyl-1,3-propandionato-κ²O,O')Rhodium(1) [Rh(bfcm)(cod)],
(6) (η⁴-1,5-cyclooctadien)(1-ferrocenyl-1,3-butandionato-κ²O,O')Rhodium(1), [Rh(fca)(cod)],
(7) (η⁴-1,5-cyclooctadien)(1-ferrocenyl-4,4,4-trichloro-1,3-butandionato-κ²O,O')Rhodium(1), [Rh(fctca)(cod)],
(8) (η⁴-1,5-cyclooctadien)(1-ferrocenyl-4,4,4-trifluooro-1,3-butandionato-κ²O,O')Rhodium(1), [Rh(fctfa)(cod)],
(9) Diferrocenoylmethan, (Hdfcm),
(10) Ferrocenoyltrifluoroaceton, (Hfctfa),
(11) (η⁴-1,5-cyclooctadien)(1-ferrocenyl-4,4,4-trifluoro-1,3-butandionato-κ²O,O')Iridium(1), [Ir(fctfa)(cod)],
(12) (η⁴-1,5-cyclooctadien)(1-ferrocenyl-1,3-butandionato-κ²O,O')Iridium(1), [Ir(fca)(cod)],
(13) (η⁴-1,5-cyclooctadien)(1-ferrocenyl-3-phenyl-1,3-propandionato-κ²O,O')Iridium(1), [Ir(bfcm)(cod)],
(14) Benzoylferrocenoylmethan, (Hbfcm),
(15) Ferrocenoylaceton, (Hfca),
(16) (η⁴-1,5-cyclooctadien)(1-ferrocenyl-1,3-propandionato-κ²O,O')Iridium(1), [Ir(fch)(cod)].
(17) (η⁴-1,5-cyclooctadien)(1-ruthenocenyl-1,3-ropandionato-κ²O,O')Rhodium(1), [Rh(RcH)(cod)],
(18) (η⁴-1,5-cyclooctadien)(1,3-diruthenocenyl-1,3-propandionato-κ²O,O')Rhodium(1) [Rh(drcm)(cod)],
(19) (η⁴-1,5-cyclooctadien)(1-ruthenocenyl-3-phenyl-1,3-propandionato-κ²O,O')Rhodium(1) [Rh(brcm)(cod)],
(20) (η⁴-1,5-cyclooctadien)(1-ruthenocenyl-1,3-butandionato-κ²O,O')Rhodium(1), [Rh(rca)(cod)],
(21) (η⁴-1,5-cyclooctadien)(1-ruthenocenyl-4,4,4-trichloro-1,3-butandionato-κ²O,O')Rhodium(1), [Rh(rctca)(cod)],
(22) (η⁴-1,5-cyclooctadien)(1-ruthenocenyl-4,4,4-trifluooro-1,3-butandionato-κ²O,O')Rhodium(1), [Rh(rctfa)(cod)],
(23) (η⁴-1,5-cyclooctadien)(1-osmocenyl-1,3-propandionato-κ²O,O')Rhodium(1), [Rh(OcH)(cod)],
(24) (η⁴-1,5-cyclooctadien)(1,3-diosmocenyl-1,3-propandionato-κ²O,O')Rhodium(1) [Rh(docm)(cod)],
(25) (η⁴-1,5-cyclooctadien)(1-osmocenyl-3-phenyl-1,3-propandionato-κ²O,O')Rhodium(1) [Rh(bocm)(cod)],
(26) (η⁴-1,5-cyclooctadien)(1-osmocenyl-1,3-butandionato-κ²O,O')Rhodium(1), [Rh(oca)(cod)],
(27) (η⁴-1,5-cyclooctadien)(1-osmocenyl-4,4,4-trichloro-1,3-butandionato-κ²O,O')Rhodium(1), [Rh(octca)(cod)],
(28) (η⁴-1,5-cyclooctadien)(1-osmocenyl-4,4,4-trifluooro-1,3-butandionato-κ²O,O')Rhodium(1), [Rh(octfa)(cod)],
(29) (η⁴-1,5-cyclooctadien)(1-ferrocenyl-3-ruthenocenyl-1,3-propandionato-κ²O,O')Rhodium(1) [Rh(fcrcm)(cod)],
(30) (η⁴-1,5-cyclooctadien)(1-ferrocenyl-3-osmocenyl-1,3-propandionato-ê²O,O=)rhodium(1) [Rh(fcocm)(cod)],
(31) (η⁴-1,5-cyclooctadien)(1-osmocenyl-3-ruthenocenyl-1,3-propandionato-κ²O,O')Rhodium(1) [Rh(ocrcm)(cod)],
(32) 1-ruthenocenyl-1,3-propandion = HRcH,
(33) 1,3-diruthenocenyl-1,3-propandion = Hdrcm,
(34) 1-ruthenocenyl-3-phenyl-1,3-propandion = Hbrcm,
(35) Ruthenocenoylaceton = 1-ruthenocenyl-1,3-butandion = Hrca,
(36) Ruthenocenoyltrichloroaceton = 1-ruthenocenyl-4,4,4-trichloro-1,3-butandion = Hrctca,
(37) Ruthenocenoyltrifluoroaceton = 1-ruthenocenyl-4,4,4-trifluoro-1,3-butandion = Hrctfa,
(38) 1-osmocenyl-1,3-propandion = HOcH
(39) 1,3-diosmocenyl -1,3-propanedione = Hdocm
(40) 1-osmocenyl-3-phenyl-1,3-propandion = Hbocm,
(41) Osmocenoylaceton = 1-osmocenyl-1,3-butandion = Hoca,
(42) Osmocenoyltrichloroaceton = 1-osmocenyl-4,4,4-trichloro-1,3-butandion = Hoctca,
(43) Osmocenoyltrifluoroaceton = 1-osmocenyl-4,4,4-trifluoro-1,3-butandion = Hoctfa,
(44) Ferrocenoylosmocenoylmethan = 1 ferrocenyl-3-osmocenyl-1,3-propandion = Hfcocm,
(45) Osmocenoylruthenocenoylmethan = 1-osmocenyl-3-ruthenocenyl-1,3-propandion = Hrcocm,
(46) Ferrocenoylruthenocenoylmethan = 1-ferrocenyl-3-ruthenocenyl-1,3-propandion = Hfcrcm,
(47) (η⁴-1,5-cyclooctadien)(1-ferrocenyl-3-ruthenocenyl-1,3-propandionato-κ²O,O')Rhodium(1) [Rh(fcrcm)(cod)],
(48) (η⁴-1,5-cyclooctadien)(1-ferrocenyl-3-osmocenyl-1,3-propandionato-κ²O,O')Rhodium(1) [Rh(fcocm)(cod)]

15. Verbindung, welche
(a) ausgewählt wird aus Metallocenyl β-Diketonen der allgemeinen Formel Mc-CO-CZ₁Z₂-CO-R, in welcher Mc ausgewählt wird aus Fc (Ferrocenyl), Rc (Ruthenocenyl) und Oc (Osmocenyl), R ist H, Alkyl, Haloalkyl, Mc oder Aryl und Z₁ und Z₂ unabhängig voneinander sind H, Alkyl, Aryl oder ein substituiertes Alkyl oder Ferrocenyl, oder
(b) ausgewählt wird aus den Enolformen der β-Diketone und der Metallkomplexe der β-Diketone der allgemeinen Formel M(β-Diketonato)A¹, M(β-Diketonato)A¹A², M(β-Diketonato)A¹B¹B², M(β-Diketonato)B¹B² und M(β-Diketonato) B¹B²B³B⁴, in welchen
β-Diketonato ist Mc-CO-CZ₁Z₂-CO-R, wie oben beschrieben, M ausgewählt wird aus Rh und Ir,
A¹ und A² gleich oder unterschiedlich sind und ausgewählt werden aus zyklischen Dienen mit 6 - 8 Kohlenstoffen oder linearen Alkenen mit 2 - 7 Kohlenstoffen
B¹B², B³ und B⁴ gleich oder unterschiedlich sind und ausgewählt werden aus CO, P(R¹R²R³), P(OR¹)(OR²)(OR³), R⁴ und X, in welchen R¹, R², R³ und R⁴ gleich oder unterschiedlich sind und unabhängig voneinander ausgewählt werden aus Alkyl, Phenyl und Ferrocenyl und X ein Halid oder ein Pseudohalid ist, mit dem Vorbehalt, dass die Verbindung nicht sein kann
(1) Ferrocenoyltrichloroaceton, (Hfctca),
(2) (η⁴-1,5-cyclooctadien)(1,3-diferrocenyl-1,3-propandionato-κ²O,O')Rhodium(1) [Rh(dfcm)(cod)],
(3) (η⁴-1,5-cyclooctadien)(1-ferrocenyl-3-phenyl-1,3-propandionato-κ²O,O')Rhodium(1) [Rh(bfcm)(cod)],
(4) (η⁴-1,5-cyclooctadien)(1-ferrocenyl-1,3-butandionato-κ²O,O')Rhodium(1), [Rh(fca)(cod)],
(5) (η⁴-1,5-cyclooctadien)(1-ferrocenyl-4,4,4-trichloro-1,3-butandionato-κ²O,O')Rhodium(1), [Rh(fctca)(cod)],
(6) (η⁴-1,5-cyclooctadien)(1-ferrocenyl-4,4,4-trifluoro-1,3-butandionato-κ²O,O')Rhodium(1), [Rh(fctfa)(cod)],
(7) Diferrocenoylmethan, (Hdfcm),
(8) Ferrocenoyltrifluoroaceton, (Hfctfa),
(9) Benzoylferrocenoylmethan, (Hbfcm),
(10) Ferrocenoylaceton, (Hfca),
(11) (η⁴-1,5-cyclooctadien)(1,3-pentandionato-κ²O,O')Rhodium(1) [Rh(acac)(cod)],
(12) (η²-1,2-ethen)₂(1-ferrocenyl-1,3-butandionato-κ²O,O')Rhodium(1),[Rh(fca)(CH₂CH₂)₂],
(13) Ferrocenoylacetaldehyd

16. Verbindung nach Anspruch 15, in welcher Z₁ und Z₂ ausgewählt werden aus Haloalkyl und Benzyl.

17. Verwendung nach Anspruch 15 oder Anspruch 16, in welcher R ausgewählt wird aus CF₃, CCl₃, CH₃, H, Ph (Phenyl) und Mc.

18. Verwendung nach einem der Ansprüche 15 bis 17 einschließlich, in welcher das Halid ausgewählt wird aus F, Cl, Br und J.

19. Verwendung nach einem der Ansprüche 15 bis 18 einschließlich, in welcher das Pseudohalid ausgewählt wird aus N₃, NCO und SCN.

20. Verwendung nach einem der Ansprüche 15 bis 19 einschließlich, in welcher die Verbindung ausgewählt wird aus der Gruppe bestehend aus [M(β-Diketonato)(cod)], [M(β-Diketonato)(CO)₂], [M(β-Diketonato)(CO)(PR²₃)], [M(β-Diketonato){P-(OR²)₃}₂] [M(β-Diketonato)(CO)₂(R³)(X)] oder seines Acylisomers, [M(β-Diketonato)(CO)(COR³)(X)], [M(β-Diketonato)(CO)(PR²₃)(R³)(X)] oder seines Acylisomers, [M(β-Diketonato)(PR²₃)(COR³)(X)], [M(β-Diketonato){P-(OR²)₃}₂(R³)(X)] und [M(β-Diketonato)(cod)R³)(X)], in welcher M ist Rh oder Ir, cod ist 1,5-cyclooctadien, (β-Diketonato) ist (McCOCHCOR), in welchen R ausgewählt wird aus CF₃, CCl₃, CH₃, H, Phenyl und Mc, R² ist Alkyl, Phenyl, Ferrocenyl und Kombinationen davon, R³ ist Alkyl, Phenyl oder Ferrocenyl und X ein Halid oder Pseudohalid ist.

21. Verbindung nach einem der Ansprüche 15 bis 20 einschließlich, in welcher ausgewählt wird aus der Gruppe bestehend den folgenden Verbindungen 1 - 37:
(1) (η⁴-1,5-cyclooctadien)(1-ferrocenyl-1,3-propandionato-(4) κ²O,O')Rhodium(1), [Rh(FcH)(cod)],
(2) η⁴-1,5-cyclooctadien)(1-ferrocenyl-4,4,4-trifluoro-1,3-butandionato-κ²O,O')Iridium(1), [Ir(fctfa)(cod)],
(3) η⁴-1,5-cyclooctadien)(1-ferrocenyl-1,3-butandionato-κ²O,O')Iridium(1), [Ir(fca)(cod)],
(4) (η⁴-1,5-cyclooctadien)(1-ferrocenyl-3-phenyl-1,3-propandionato-κ²O,O')Iridium(1), [Ir(bfcm)(cod)],
(5) (η⁴-1,5-cyclooctadien)(1-ferrocenyl-1,3-propandionato-κ²O,O')Iridium(1), [Ir(fch)(cod)].
(6) (η⁴-1,5-cyclooctadien)(1-ruthenocenyl-1,3-propandionato-κ²O,O')Rhodium(1), [Rh(RcH)(cod)],
(7) (η⁴-1,5-cyclooctadien)(1,3-diruthenocenyl-1,3-propandionato-κ²O,O')Rhodium(1) [Rh(drcm)(cod)],
(8) (η⁴-1,5-cyclooctadien)(1-ruthenocenyl-3-phenyl-1,3-propandionato-κ²O,O')Rhodium(1) [Rh(brcm)(cod)],
(9) (η⁴-1,5-cyclooctadien)(1-ruthenocenyl-1,3-butandionato-κ²O,O')Rhodium(1), [Rh(rca)(cod)],
(10) (η⁴-1,5-cyclooctadien)(1-ruthenocenyl-4,4,4-trichloro-1,3-butandionato-κ²O,O')Rhodium(1), [Rh(rctca)(cod)],
(11) (η⁴-1,5-cyclooctadien)(1-ruthenocenyl-4,4,4-trifluoro-1,3-butandionato-κ²O,O')Rhodium(1), [Rh(rctfa)(cod)],
(12) (η⁴-1,5-cyclooctadien)(1-osmocenyl-1,3-propandionato-κ²O,O')Rhodium(1), [Rh(OcH)(cod)],
(13) (η⁴-1,5-cyclooctadien)(1,3-diosmocenyl-1,3-propandionato-κ²O,O')Rhodium(1) [Rh(docm)(cod)],
(14) (η⁴-1,5-cyclooctadien)(1-osmocenyl-3-phenyl-1,3-propandionato-κ²O,O')Rhodium(1) [Rh(bocm)(cod)],
(15) (η⁴-1,5-cyclooctadien)(1-osmocenyl-1,3-butandionato-κ²O,O')Rhodium(1), [Rh(oca)(cod)],
(16) (η⁴-1,5-cyclooctadien)(1-osmocenyl-4,4,4-trichloro-1,3-butandionato-κ²O,O')Rhodium(1), [Rh(octca)(cod)],
(17) (η⁴-1,5-cyclooctadien)(1-osmocenyl-4,4,4-trifluooro-1,3-butandionato-κ²O,O')Rhodium(1), [Rh(octfa)(cod)],
(18) (η⁴-1,5-cyclooctadien)(1-ferrocenyl-3-ruthenocenyl-1,3-propandionato-κ²O,O')Rhodium(1) [Rh(fcrcm)(cod)],
(19) (η⁴-1,5-cyclooctadien)(1-ferrocenyl-3-osmocenyl-1,3-propandionato-κ²O,O')Rhodium(1) [Rh(fcocm)(cod)],
(20) (η⁴-1,5-cyclooctadien)(1-osmocenyl-3-ruthenocenyl-1,3-propandionato-κ²O,O')Rhodium(1) [Rh(ocrcm)(cod)],
(21) 1-ruthenocenyl-1,3-propandion = HRcH,
(22) 1,3-diruthenocenyl-1,3-propandion = Hdrcm,
(23) 1-ruthenocenyl-3-phenyl-1,3-propandion = Hbrcm,
(24) Ruthenocenoylaceton = 1-ruthenocenyl-1,3-butandion = Hrca,
(25) Ruthenocenoyltrichloroaceton = 1-ruthenocenyl-4,4,4-trichloro-1,3-butandion = Hrctca,
(26) Ruthenocenoyltrifluoroaceton = 1-ruthenocenyl-4,4,4-trifluoro-1,3-butandion = Hrctfa,
(27) 1-osmocenyl-1,3-propandion = HOcH
(28) 1,3-diosmocenyl -1,3-propandion = Hdocm
(29) 1-osmocenyl-3-phenyl-1,3-propandion = Hbocm,
(30) Osmocenoylaceton = 1-osmocenyl-1,3-butandion = Hoca,
(31) Osmocenoyltrichloroaceton = 1-osmocenyl-4,4,4-trichloro-1,3-butandion = Hoctca,
(32) Osmocenoyltrifluoroaceton = 1-osmocenyl-4,4,4-trifluoro-1,3-butandion = Hoctfa,
(33) Ferrocenoylosmocenoylmethan = 1 ferrocenyl-3-osmocenyl-1,3-propandion = Hfcocm,
(34) Osmocenoylruthenocenoylmethan = 1-osmocenyl-3-ruthenocenyl-1,3-propanedion = Hrcocm,
(35) Ferrocenoylruthenocenoylmethan = 1-ferrocenyl-3-ruthenocenyl-1,3-propanedion = Hfcrcm,
(36) (η⁴-1,5-cyclooctadien)(1-ferrocenyl-3-ruthenocenyl-1,3-propandionato-κ²O,O')Rhodium(1) [Rh(fcrcm)(cod)],
(37) (η⁴-1,5-cyclooctadien)(1-ferrocenyl-3-osmocenyl-1,3-propandionato-κ²O,O')Rhodium(1) [Rh(fcocm)(cod)]

## Revendications

1. Utilisation d'une substance ou composition dans la préparation d'un médicament pour le traitement chimiothérapeutique du cancer, la substance ou composition comprenant :
(a) au moins un composé qui est choisi parmi des métallocényl-β-dicétones de formule générale Mc-CO-CZ₁Z₂-CO-R, dans laquelle Mc est choisi entre Fc (ferrocényle), Rc (ruthénocényle) et Oc (osmocényle), R représente H, un groupe alkyle, haloalkyle, Mc ou aryle, et Z₁ et Z₂ représentent indépendamment H, un groupe alkyle, aryle ou alkyle substitué ou ferrocényle, ou
(b) au moins un composé qui est choisi parmi les formes énol des β-dicétones et des complexes métalliques de β-dicétones de formules générales M(β-dicétonato)A¹, M(β-dicétonato)A¹A², M(β-dicétonato)A¹B¹B², M(β-dicétonato)B¹B² et M(β-dicétonato)B¹B²B³B⁴, dans lesquelles
β-dicétonato est un groupe Mc-CO-CZ₁Z₂-CO-R tel que décrit ci-dessus,
M est choisi entre Rh et Ir,
A¹ et A² sont identiques ou différents et sont choisis entre des diènes cycliques ayant 6-8 atomes de carbone et des alcènes linéaires ayant 2-7 atomes de carbone,
B¹, B², B³ et B⁴ sont identiques ou différents et sont choisis entre CO, P(R¹R²R³), P(OR¹)(OR²)(OR³), R⁴ et X, où R¹, R², R³ et R⁴ sont identiques ou différents et sont choisis indépendamment entre des groupes alkyle, phényle et ferrocényle, et X est un halogénure ou un pseudohalogénure.

2. Utilisation selon la revendication 1, dans laquelle Z₁ et Z₂ sont choisis entre les groupes haloalkyle et benzyle.

3. Utilisation selon la revendication 1 ou 2, dans laquelle R est choisi entre CF₃, CCl₃, CH₃, H, Ph (phényle) et Mc.

4. Utilisation selon l'une quelconque des revendications 1 à 3 comprise, dans laquelle l'halogénure est choisi entre F, Cl, Br et I.

5. Utilisation suivant l'une quelconque des revendications 1 à 4 comprise, dans laquelle le pseudohalogénure est choisi entre N₃, NCO et SCN.

6. Utilisation suivant l'une quelconque des revendications 1 à 5 comprise, dans laquelle le composé est choisi dans le groupe constitué par [M(β-dicétonato)(cod)], [M(β-dicétonato)(CO₂)], [M(β-dicétonato)(CO)-(PR²₃)], [M(β-dicétonato){P-(OR²)₃}₂][M(β-dicétonato)(CO₂)(R₃)(X)] ou son isomère acyle, [M(β-dicétonato)(CO)(COR³)(X)], [M(β-dicétonato)(CO)(PR²₃)(R³)(X)] ou son isomère acyle, [M(β-dicétonato)(PR²₃)(COR³)(X)], [M(β-dicétonato){P-(OR²)₃}₂(R³)(X)] et [M(β-dicétonato)(cod)(R³)(X)], où M représente Rh ou Ir, cod est un groupe 1,5-cyclooctadiène, (β-dicétonato) représente un groupe (McCOCHCOR) où R est choisi entre CF₃, CCl₃, CH₃, H, phényle et Mc, R² est un groupe alkyle, phényle, ferrocényle et leurs combinaisons, R³ est un groupe alkyle, phényle ou ferrocényle, et X est un halogénure ou un pseudohalogénure.

7. Utilisation selon l'une quelconque des revendications 1 à 6 comprise, dans laquelle le composé est choisi parmi les composés 1-38 suivants :
(1) ferrocénoylacétaldéhyde, (Hfch),
(2) (η⁴-1,5-cyclooctadiène)(1-ferrocényl-1,3-propanedionato-κ²O,O')rhodium(1), [Rh(FcH)(cod)],
(3) (η⁴-1,5-cyclooctadiène)(1-ferrocényl-4,4,4-trifluoro-1,3-butane-dionato-κ²O,O')iridium(1), [Ir(fctfa)(cod)],
(4) (η⁴-1,5-cyclooctadiène)(1-ferrocényl-1,3-butanedionato-κ²O,O')-iridium(1), [Ir(fca)(cod)],
(5) (η⁴-1,5-cyclooctadiène)(1-ferrocényl-3-phényl-1,3-propane-dionato-κ²O,O')iridium(1), [Ir(bfcm)(cod)],
(6) (η⁴-1,5-cyclooctadiène)(1-ferrocényl-1,3-propanedionato-κ²O,O')iridium(1), [Ir(fch)(cod)],
(7) (η⁴-1,5-cyclooctadiène)(1-ruthénocényl-1,3-propanedionato-κ²O,O')rhodium(1), [Rh(RcH)(cod)],
(8) (η⁴-1,5-cyclooctadiène)(1,3-diruthénocényl-1,3-propanedionato-κ²O,O')rhodium(1) [Rh(drcm)(cod)],
(9) (η⁴-1,5-cyclooctadiène)(1-ruthénocényl-3-phényl-1,3-propane-dionato-κ²O,O')rhodium(1) [Rh(brcm)(cod)],
(10) (η⁴-1,5-cyclooctadiène)(1-ruthénocényl-1,3-butanedionato-κ²O,O')rhodium(1), [Rh(rca)(cod)],
(11) (η⁴-1,5-cyclooctadiène)(1-ruthénocényl-4,4,4-trichloro-1,3-butanedionato-κ²O,O')rhodium(1), [Rh(rctca)(cod)],
(12) (η⁴-1,5-cyclooctadiène)(1-ruthénocényl-4,4,4-trifluoro-1,3-butanedionato-κ²O,O')rhodium(1), [Rh(rctfa)(cod)],
(13) (η⁴-1,5-cyclooctadiène)(1-osmocényl-1,3-propanedionato-κ²O,O')rhodium(1), [Rh(OcH)(cod)],
(14) (η⁴-1,5-cyclooctadiène)(1,3-diosmocényl-1,3-propanedionato-κ²O,O')rhodium(1), [Rh(docm)(cod)],
(15) (η⁴-1,5-cyclooctadiène)(1-osmocényl-3-phényl-1,3-propanedionato-κ²O,O')rhodium(1), [Rh(bocm)(cod)],
(16) (η⁴-1,5-cyclooctadiène)(1-osmocényl-1,3-butanedionato-κ²O,O')rhodium(1), [Rh(oca)(cod)],
(17) (η⁴-1,5-cyclooctadiène)(1-osmocényl-4,4,4-trichloro-1,3-butanedionato-κ²O,O')rhodium(1), [Rh(octca)(cod)],
(18) (η⁴-1,5-cyclooctadiène)(1-osmocényl-4,4,4-trifluoro-1,3-butanedionato-κ²O,O')rhodium(1), [Rh(octfa)(cod)],
(19) (η⁴-1,5-cyclooctadiène)(1-ferrocényl-3-ruthénocényl-1,3-propanedionato-κ²O,O')rhodium(1), [Rh(fcrcm)(cod)],
(20) (η⁴-1,5-cydooctadiène)(1-ferrocényl-3-osmocényl-1,3-propanedionato-κ²O,O')rhodium(1), [Rh(fcocm)(cod)],
(21) (η⁴-1,5-cyclooctadiène)(1-osmocényl-3-ruthénocényl-1,3-propanedionato-κ²O,O')rhodium(1), [Rh(ocrcm)(cod)],
(22) 1-ruthénocényl-1,3-propanedione = HRcH,
(23) 1,3-diruthénocényl-1,3-propanedione = Hdrcm,
(24) 1-ruthénocényl-3-phényl-1,3-propanedione = Hbrcm,
(25) ruthénocénoylacétone = 1-ruthénocényl-1,3-butanedione = Hrca,
(26) ruthénocénoyltrichloroacétone = 1-ruthénocényl-4,4,4-trichloro-1,3-butanedione = Hrctca,
(27) ruthénocénoyltrifluoroacétone = 1-ruthénocényl-4,4,4-trifluoro-1,3-butanedione = Hrctfa,
(28) 1-osmocényl-1,3-propanedione = HOcH,
(29) 1,3-diosmocényl-1,3-propanedione : Hdocm,
(30) 1-osmocényl-3-phényl-1,3-propanedione = Hbocm,
(31) osmocénoylacétone = 1-osmocényl-1,3-butanedione = Hoca,
(32) osmocénoyltrichloroacétone = 1-osmocényl-4,4,4-trichloro-1,3-butanedione = Hoctca,
(33) osmocénoyltrifluoroacétone = 1-osmocényl-4,4,4-trifluoro-1,3-butanedione = Hoctfa,
(34) ferrocénoylosmocénoylméthane = 1-ferrocényl-3-osmocényl-1,3-propanedione = Hfcocm,
(35) osmocénoylruthénocénoylméthane = 1-osmocényl-3-ruthéno-cényl-1,3-propanedione = Hrcocm,
(36) ferrocénoylruthénocénoylméthane = 1-ferrocényl-3-ruthéno-cényl-1,3-propanedione = Hfcrcm,
(37) (η⁴-1,5-cyclooctadiène)(1-ferrocényl-3-ruthénocényl-1,3-propanedionato-κ²O,O')rhodium(1), [Rh(fcrcm)(cod)],
(38) (η⁴-1,5-cyclooctadiène)(1-ferrocényl-3-osmocényl-1,3-propane-dionato-κ²O,O')rhodium(1), [Rh(fcocm)(cod)].

8. Utilisation d'une substance ou composition pour la préparation d'un médicament à utiliser dans la sensibilisation de cellules à un rayonnement, la substance ou composition comprenant :
(a) au moins un composé qui est choisi parmi des métallocényl-β-dicétones de formule générale Mc-CO-CZ₁Z₂-CO-R, dans laquelle Mc est choisi entre Fc (ferrocényle), Rc (ruthénocényle) et Oc (osmocényle), R représente H, un groupe alkyle, haloalkyle, Mc ou aryle, et Z₁ et Z₂ représentent indépendamment H, un groupe alkyle, aryle ou alkyle substitué ou ferrocényle, ou
(b) au moins un composé qui est choisi parmi les formes énol des β-dicétones et des complexes métalliques de β-dicétones de formules générales M(β-dicétonato)A¹, M(β-dicétonato)A¹A², M(β-dicétonato)A¹B¹B², M(β-dicétonato)B¹B² et M(β-dicétonato)B¹B²B³B⁴, dans lesquelles
β-dicétonato est un groupe Mc-CO-CZ₁Z₂-CO-R tel que décrit ci-dessus,
M est choisi entre Rh et Ir,
A¹ et A² sont identiques ou différents et sont choisis entre des diènes cycliques ayant 6-8 atomes de carbone et des alcènes linéaires ayant 2-7 atomes de carbone,
B¹, B², B³ et B⁴ sont identiques ou différents et sont choisis entre CO, P(R¹R²R³), P(OR¹)(OR²)(OR³), R⁴ et X, où R¹, R², R³ et R⁴ sont identiques ou différents et sont choisis indépendamment entre des groupes alkyle, phényle et ferrocényle, et X est un halogénure ou un pseudohalogénure.

9. Utilisation selon la revendication 8, dans laquelle Z₁ et Z₂ sont choisis parmi les groupes haloalkyle et benzyle.

10. Utilisation selon la revendication 8 ou 9, dans laquelle R est choisi entre CF₃, CCl₃, CH₃, H, Ph (phényle) et Mc.

11. Utilisation selon l'une quelconque des revendications 8 à 10 comprise, dans laquelle l'halogénure est choisi entre F, Cl, Br et I.

12. Utilisation selon l'une quelconque des revendications 8 à 11 comprise, dans laquelle le pseudohalogénure est choisi entre N₃, NCO et SCN.

13. Utilisation selon l'une quelconque des revendications 8 à 12 comprise, dans laquelle le composé est choisi dans le groupe constitué par [M(β-dicétonato)(cod)], [M(β-dicétonato)(CO₂)], [M(β-dicétonato)(CO)-(PR²₃)], [M(β-dicétonato){P-(OR²)₃}₂][M(β-dicétonato)(CO₂)(R₃)(X)] ou son isomère acyle, [M(β-dicétonato)(CO)(COR³)(X)], [M(β-dicétonato)(CO)(PR²₃)(R³)(X)] ou son isomère acyle, [M(β-dicétonato)(PR²₃)(COR³)(X)], [M(β-dicétonato){P-(OR²)₃}₂(R³)(X)] et [M(β-dicétonato)(cod)(R³)(X)], où M représente Rh ou Ir, cod est un groupe 1,5-cyclooctadiène, (β-dicétonato) représente un groupe (McCOCHCOR) dans lequel R est choisi entre CF₃, CCl₃, CH₃, H, phényle et Mc, R² est un groupe alkyle, phényle, ferrocényle et leurs combinaisons, R³ est un groupe alkyle, phényle ou ferrocényle, et X est un halogénure ou un pseudohalogénure.

14. Utilisation selon l'une quelconque des revendications 8 à 13 comprise, dans laquelle le composé est choisi parmi les composés 1-48 suivants :
(1) ferrocénoylacétaldéhyde, (Hfch),
(2) ferrocénoyltrichloroacétone (Hfctca),
(3) (η⁴-1,5-cyclooctadiène)(1-ferrocényl-1,3-propanedionato-κ²O,O')rhodium(1), [Rh(FcH)(cod)],
(4) (η⁴-1,5-cyclooctadiène)(1,3-diferrocényl-1,3-propanedionato-κ²O,O')rhodium(1), [Rh(dfcm)(cod)],
(5) (η⁴-1,5-cyclooctadiène)(1-ferrocényl-3-phényl-1,3-propane-dionato-κ²O,O')rhodium(1), [Rh(bfcm)(cod)],
(6) (η⁴-1,5-cyclooctadiène)(1-ferrocényl-1,3-butanedionato-κ²O,O')rhodium(1), [Rh(fca)(cod)],
(7) (η⁴-1,5-cyclooctadiène)(1-ferrocényl-4,4,4-trichloro-1,3-butanedionato-κ²O,O')rhodium(1), [Rh(fctca)(cod)],
(8) (η⁴-1,5-cyclooctadiène)(1-ferrocényl-4,4,4-trifluoro-1,3-butanedionato-κ²O,O')rhodium(1) [Rh(fctfa)(cod)],
(9) diferrocénoylméthane (Hdfcm),
(10) ferrocénoyltrifluoroacétone (Hfctfa),
(11) (η⁴-1,5-cyclooctadiène)(1-ferrocényl-4,4,4-trifluoro-1,3-butane-dionato-κ²O,O')iridium(1) [Ir(fctfa)(cod)],
(12) (η⁴-1,5-cyclooctadiène)(1-ferrocényl-1,3-butanedionato-κ²O,O')iridium(1), [Ir(fca)(cod)],
(13) (η⁴-1,5-cyclooctadiène)(1-ferrocényl-3-phényl-1,3-propanedionato-κ²O,O')iridium(1), [Ir(bfcm)(cod)],
(14) benzoylferrocénoylméthane, (Hbfcm),
(15) ferrocénoylacétone, (Hfca),
(16) (η⁴-1,5-cyclooctadiène)(1-ferrocényl-1,3-propanedionato-κ²O,O')iridium(1), [Ir(fch)(cod)],
(17) (η⁴-1,5-cyclooctadiène)(1-ruthénocényl-1,3-propanedionato-κ²O,O')rhodium(1), [Rh(RcH)(cod)],
(18) (η⁴-1,5-cyclooctadiène)(1,3-diruthénocényl-1,3-propanedionato-κ²O,O')rhodium(1), [Rh(drcm)(cod)],
(19) (η⁴-1,5-cyclooctadiène)(1-ruthénocényl-3-phényl-1,3-propanedionato-κ²O,O')rhodium(1), [Rh(brcm)(cod)],
(20) (η⁴-1,5-cyclooctadiène)(1-ruthénocényl-1,3-butanedionato-κ²O,O')rhodium(1), [Rh(rca)(cod)],
(21) (η⁴-1,5-cyclooctadiène)(1-ruthénocényl-4,4,4-trichloro-1,3-butanedionato-κ²O,O')rhodium(1), [Rh(rctca)(cod)],
(22) (η⁴-1,5-cyclooctadiène)(1-ruthénocényl-4,4,4-trifluoro-1,3-butanedionato-κ²O,O')rhodium(1), [Rh(rctfa)(cod)],
(23) (η⁴-1,5-cyclooctadiène)(1-osmocényl-1,3-propanedionato-κ²O,O')rhodium(1), [Rh(OcH)(cod)],
(24) (η⁴-1,5-cyclooctadiène)(1,3-diosmocényl-1,3-propanedionato-κ²O,O')rhodium(1), [Rh(docm)(cod)],
(25) (η⁴-1,5-cyclooctadiène)(1-osmocényl-3-phényl-1,3-propanedionato-κ²O,O')rhodium(1), [Rh(bocm)(cod)],
(26) (η⁴-1,5-cyclooctadiène)(1-osmocényl-1,3-butanedionato-κ²O,O')rhodium(1), [Rh(oca)(cod)],
(27) (η⁴-1,5-cyclooctadiène)(1-osmocényl-4,4,4-trichloro-1,3-butanedionato-κ²O,O')rhodium(1), [Rh(octca)(cod)],
(28) (η⁴-1,5-cyclooctadiène)(1-osmocényl-4,4,4-trifluoro-1,3-butanedionato-κ²O,O')rhodium(1), [Rh(octfa)(cod)],
(29) (η⁴-1,5-cyclooctadiène)(1-ferrocényl-3-ruthénocényl-1,3-propanedionato-κ²O,O')rhodium(1), [Rh(fcrcm)(cod)],
(30) (η⁴-1,5-cyclooctadiène)(1-ferrocényl-3-osmocényl-1,3-propanedionato-κ²O,O')rhodium(1), [Rh(fcocm)(cod)],
(31) (η⁴-1,5-cyclooctadiène)(1-osmocényl-3-ruthénocényl-1,3-propanedionato-κ²O,O')rhodium(1), [Rh(ocrcm)(cod)],
(32) 1-ruthénocényl-1,3-propanedione = HRcH,
(33) 1,3-diruthénocényl-1,3-propanedione = Hdrcm,
(34) 1-ruthénocényl-3-phényl-1,3-propanedione = Hbrcm,
(35) ruthénocénoylacétone = 1-ruthénocényl-1,3-butanedione = Hrca,
(36) ruthénocénoyltrichloroacétone = 1-ruthénocényl-4,4,4-trichloro-1,3-butanedione = Hrctca,
(37) ruthénocénoyltrifluoroacétone = 1-ruthénocényl-4,4,4-trifluoro-1,3-butanedione = Hrctfa,
(38) 1-osmocényl-1,3-propanedione = HOcH,
(39) 1,3-diosmocényl-1,3-propanedione : Hdocm,
(40) 1-osmocényl-3-phényl-1,3-propanedione = Hbocm,
(41) osmocénoylacétone = 1-osmocényl-1,3-butanedione = Hoca,
(42) osmocénoyltrichloroacétone = 1-osmocényl-4,4,4-trichloro-1,3-butanedione = Hoctca,
(43) osmocénoyltrifluoroacétone = 1-osmocényl-4,4,4-trifluoro-1,3-butanedione = Hoctfa,
(44) ferrocénoylosmocénoylméthane = 1-ferrocényl-3-osmocényl-1,3-propanedione = Hfcocm,
(45) osmocénoylruthénocénoylméthane = 1-osmocényl-3-ruthéno-cényl-1,3-propanedione = Hrcocm,
(46) ferrocénoylruthénocénoylméthane = 1-ferrocényl-3-ruthéno-cényl-1,3-propanedione = Hfcrcm,
(47) (η⁴-1,5-cyclooctadiène)(1-ferrocényl-3-ruthénocényl-1,3-propanedionato-κ²O,O')rhodium(1), [Rh(fcrcm)(cod)],
(48) (η⁴-1,5-cyclooctadiène)(1-ferrocényl-3-osmocényl-1,3-propane-dionato-κ²O,O')rhodium(1), [Rh(fcocm)(cod)].

15. Composé qui est :
(a) choisi entre des métallocényl-β-dicétones de formule générale Mc-CO-CZ₁Z₂-CO-R, dans laquelle Mc est choisi entre Fc (ferrocényle), Rc (ruthénocényle) et Oc (osmocényle), R représente H, un groupe alkyle, haloalkyle, Mc ou aryle, et Z₁ et Z₂ représentent indépendamment H, un groupe alkyle, aryle ou alkyle substitué ou ferrocényle, ou
(b) choisi parmi les formes énol des β-dicétones et des complexes métalliques de β-dicétones de formules générales M(β-dicétonato)A¹, M(β-dicétonato)A¹A², M(β-dicétonato)A¹B¹B², M(β-dicétonato)B¹B² et M(β-dicétonato)B¹B²B³B⁴, dans lesquelles
β-dicétonato est un groupe Mc-CO-CZ₁Z₂-CO-R tel que décrit ci-dessus,
M est choisi entre Rh et Ir,
A¹ et A² sont identiques ou différents et sont choisis entre des diènes cycliques ayant 6-8 atomes de carbone et des alcènes linéaires ayant 2-7 atomes de carbone,
B¹, B², B³ et B⁴ sont identiques ou différents et sont choisis entre CO, P(R¹R²R³), P(OR¹)(OR²)(OR³), R⁴ et X, où R¹, R², R³ et R⁴ sont identiques ou différents et sont choisis indépendamment entre des groupes alkyle, phényle et ferrocényle, et X est un halogénure ou un pseudohalogénure, sous réserve que le composé ne puisse pas être l'un des suivants :
(1) ferrocénoyltrichloroacétone (Hfctca),
(2) (η⁴-1,5-cyclooctadiène)(1,3-diferrocényl-1,3-propanedionato-κ²O,O')rhodium(1), [Rh(dfcm)(cod)],
(3) (η⁴-1,5-cyclooctadiène)(1-ferrocényl-3-phényl-1,3-propane-dionato-κ²O,O')rhodium(1), [Rh(bfcm)(cod)],
(4) (η⁴-1,5-cyclooctadiène)(1-ferrocényl-1,3-butanedionato-κ²O,O')rhodium(1), [Rh(fca)(cod)],
(5) (η⁴-1,5-cyclooctadiène)(1-ferrocényl-4,4,4-trichloro-1,3-butanedionato-κ²O,O')rhodium(1), [Rh(fctca)(cod)],
(6) (η⁴-1,5-cyclooctadiène)(1-ferrocényl-4,4,4-trifluoro-1,3-butanedionato-κ²O,O')rhodium(1) [Rh(fctfa)(cod)],
(7) diferrocénoylméthane (Hdfcm),
(8) ferrocénoyltrifluoroacétone (Hfctfa),
(9) benzoylferrocénoylméthane, (Hbfcm),
(10) ferrocénoylacétone, (Hfca),
(11) (η⁴-1,5-cyclooctadiène)(1,3-pentanedionato-κ²O,O')rhodium(1) [Rh(acac)(cod)],
(12) (η²-1,2-éthène)₂(1-ferrocényl-1,3-butanedionato-κ²O,O')-rhodium(1), [Rh(fca)(CH₂CH₂)₂],
(13) ferrocénoylacétaldéhyde, (Hfch).

16. Composé selon la revendication 15, dans lequel Z₁ et Z₂ sont choisis entre des groupes haloalkyle et benzyle.

17. Composé selon la revendication 15 ou 16, dans lequel R est choisi entre CF₃, CCl₃, CH₃, H, Ph (phényle) et Mc.

18. Composé selon l'une quelconque des revendications 15 à 17 comprise, dans lequel l'halogénure est F, Cl, Br ou I.

19. Composé selon l'une quelconque des revendications 15 à 18 comprise, dans lequel le pseudohalogénure est N₃, NCO ou SCN.

20. Composé selon l'une quelconque des revendications 15 à 19 comprise, qui est choisi dans le groupe constitué par [M(β-dicétonato)(cod)], [M(β-dicétonato)(CO₂)], [M(β-dicétonato)(CO)-(PR²₃)], [M(β-dicétonato){P-(OR²)₃}₂][M(β-dicétonato)(CO₂)(R₃)(X)] ou son isomère acyle, [M(β-dicétonato)(CO)(COR³)(X)], [M(β-dicétonato)(CO)(PR²₃)(X)] ou son isomère acyle, [M(β-dicétonato)(PR²₃)(COR³)(X)], [M(β-dicétonato){P-(OR²)₃}₂(R³)(X)] et [M(β-dicétonato)(cod)(R³)(X)], où M représente Rh ou Ir, cod est un groupe 1,5-cyclooctadiène, (β-dicétonato) représente le groupe (McCOCHCOR) dans lequel R est choisi entre CF₃, CCl₃, CH₃, H, phényle et Mc, R² est un groupe alkyle, phényle, ferrocényle et leurs combinaisons, R³ est un groupe alkyle, phényle ou ferrocényle, et X est un halogénure ou un pseudohalogénure.

21. Composé selon l'une quelconque des revendications 15 à 20 comprise, qui est choisi dans le groupe constitué par les composés 1 - 37 suivants :
(1) (η⁴-1,5-cyclooctadiène)(1-ferrocényl-1,3-propanedionato-κ²O,O')rhodium(1), [Rh(FcH)(cod)],
(2) (η⁴-1,5-cyclooctadiène)(1-ferrocényl-4,4,4-trifluoro-1,3-butane-dionato-κ²O,O')iridium(1), [Ir(fctfa)(cod)],
(3) (η⁴-1,5-cyclooctadiène)(1-ferrocényl-1,3-butanedionato-κ²O,O')-iridium(1), [Ir(fca)(cod)],
(4) (η⁴-1,5-cyclooctadiène)(1-ferrocényl-3-phényl-1,3-propane-dionato-κ²O,O')iridium(1), [Ir(bfcm)(cod)],
(5) (η⁴-1,5-cyclooctadiène)(1-ferrocényl-1,3-propanedionato-κ²O,O')iridium(1), [Ir(fch)(cod)],
(6) (η⁴-1,5-cyclooctadiène)(1-ruthénocényl-1,3-propanedionato-κ²O,O')rhodium(1), [Rh(RcH)(cod)],
(7) (η⁴-1,5-cyclooctadiène)(1,3-diruthénocényl-1,3-propanedionato-κ²O,O')rhodium(1) [Rh(drcm)(cod)],
(8) (η⁴-1,5-cyclooctadiène)(1-ruthénocényl-3-phényl-1,3-propane-dionato-κ²O,O')rhodium(1) [Rh(brcm)(cod)],
(9) (η⁴-1,5-cyclooctadiène)(1-ruthénocényl-1,3-butanedionato-κ²O,O')rhodium(1), [Rh(rca)(cod)],
(10) (η⁴-1,5-cyclooctadiène)(1-ruthénocényl-4,4,4-trichloro-1,3-butanedionato-κ²O,O')rhodium(1), [Rh(rctca)(cod)],
(11) (η⁴-1,5-cyclooctadiène)(1-ruthénocényl-4,4,4-trifluoro-1,3-butanedionato-κ²O,O')rhodium(1), [Rh(rctfa)(cod)],
(12) (η⁴-1,5-cyclooctadiène)(1-osmocényl-1,3-propanedionato-κ²O,O')rhodium(1), [Rh(OcH)(cod)],
(13) (η⁴-1,5-cyclooctadiène)(1,3-diosmocényl-1,3-propanedionato-κ²O,O')rhodium(1), [Rh(docm)(cod)],
(14) (η⁴-1,5-cyclooctadiène)(1-osmocényl-3-phényl-1,3-propanedionato-κ²O,O')rhodium(1), [Rh(bocm)(cod)],
(15) (η⁴-1,5-cyclooctadiène)(1-osmocényl-1,3-butanedionato-κ²O,O')rhodium(1), [Rh(oca)(cod)],
(16) (η⁴-1,5-cyclooctadiène)(1-osmocényl-4,4,4-trichloro-1,3-butanedionato-κ²O,O')rhodium(1), [Rh(octca)(cod)],
(17) (η⁴-1,5-cyclooctadiène)(1-osmocényl-4,4,4-trifluoro-1,3-butanedionato-κ²O,O')rhodium(1), [Rh(octfa)(cod)],
(18) (η⁴-1,5-cyclooctadiène)(1-ferrocényl-3-ruthénocényl-1,3-propanedionato-κ²O,O')rhodium(1), [Rh(fcrcm)(cod)],
(19) (η⁴-1,5-cyclooctadiène)(1-ferrocényl-3-osmocényl-1,3-propanedionato-κ²O,O')rhodium(1), [Rh(fcocm)(cod)],
(20) (η⁴-1,5-cyclooctadiène)(1-osmocényl-3-ruthénocényl-1,3-propanedionato-κ²O,O')rhodium(1), [Rh(ocrcm)(cod)],
(21) 1-ruthénocényl-1,3-propanedione = HRcH,
(22) 1,3-diruthénocényl-1,3-propanedione = Hdrcm,
(23) 1-ruthénocényl-3-phényl-1,3-propanedione = Hbrcm,
(24) ruthénocénoylacétone = 1-ruthénocényl-1,3-butanedione = Hrca,
(25) ruthénocénoyltrichloroacétone = 1-ruthénocényl-4,4,4-trichloro-1,3-butanedione = Hrctca,
(26) ruthénocénoyltrifluoroacétone = 1-ruthénocényl-4,4,4-trifluoro-1,3-butanedione = Hrctfa,
(27) 1-osmocényl-1,3-propanedione = HOcH,
(28) 1,3-diosmocényl-1,3-propanedione : Hdocm,
(29) 1-osmocényl-3-phényl-1,3-propanedione = Hbocm,
(30) osmocénoylacétone = 1-osmocényl-1,3-butanedione = Hoca,
(31) osmocénoyltrichloroacétone = 1-osmocényl-4,4,4-trichloro-1,3-butanedione = Hoctca,
(32) osmocénoyltrifluoroacétone = 1-osmocényl-4,4,4-trifluoro-1,3-butanedione = Hoctfa,
(33) ferrocénoylosmocénoylméthane = 1-ferrocényl-3-osmocényl-1,3-propanedione = Hfcocm,
(34) osmocénoylruthénocénoylméthane = 1-osmocényl-3-ruthéno-cényl-1,3-propanedione = Hrcocm,
(35) ferrocénoylruthénocénoylméthane = 1-ferrocényl-3-ruthéno-cényl-1,3-propanedione = Hfcrcm,
(36) (η⁴-1,5-cyclooctadiène)(1-ferrocényl-3-ruthénocényl-1,3-propanedionato-κ²O,O')rhodium(1), [Rh(fcrcm)(cod)],
(37) (η⁴-1,5-cyclooctadiène)(1-ferrocényl-3-osmocényl-1,3-propane-dionato-κ²O,O')rhodium(1), [Rh(fcocm)(cod)].
